(19) Europäisches Patentamt European Patent Office Office européen des brevets

(11) **EP 3 389 752 B1**

(12) **EUROPEAN PATENT SPECIFICATION**

(45) Date of publication and mention
of the grant of the patent:
**26.02.2025 Bulletin 2025/09**

(21) Application number: **15823836.0**

(22) Date of filing: **16.12.2015**

(51) International Patent Classification (IPC):
**A61M 16/00** (2006.01)     **A61B 5/0205** (2006.01)
**A61B 5/145** (2006.01)

(52) Cooperative Patent Classification (CPC):
(C-Sets available)
**A61M 16/021; A61M 16/0051; A61M 16/026;**
A61B 5/0205; A61B 5/14542; A61B 2505/05;
A61M 16/0045; A61M 2016/0027; A61M 2016/0036;
A61M 2230/202; A61M 2230/432          (Cont.)

(86) International application number:
**PCT/SE2015/051357**

(87) International publication number:
**WO 2017/105304 (22.06.2017 Gazette 2017/25)**

(54) **VENTILATION PATTERN FOR NON-INVASIVE DETERMINATION OF ELV, EPBF, CARDIAC OUTPUT AND/OR CO2 CONTENT IN VENOUS BLOOD**

BEATMUNGSMUSTER ZUR NICHTINVASIVEN BESTIMMUNG VON ELV, EPBF, HERZZEITVOLUMEN UND/ODER DES CO2-GEHALTS IN VENÖSEM BLUT

PROFIL DE VENTILATION POUR DÉTERMINATION NON INVASIVE D'ELV, D'EPBF, DU DÉBIT CARDIAQUE ET/OU DE LA TENEUR EN CO2 DANS LE SANG VEINEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**24.10.2018 Bulletin 2018/43**

(73) Proprietor: **Maquet Critical Care AB
171 06 Solna (SE)**

(72) Inventor: **HALLBÄCK, Magnus
182 35 Danderyd (SE)**

(74) Representative: **Zacco Sweden AB
P.O. Box 5581
Löjtnantsgatan 21
114 85 Stockholm (SE)**

(56) References cited:
**US-A1- 2002 174 866     US-A1- 2013 253 359
US-A1- 2013 253 359     US-A1- 2013 345 586
US-A1- 2013 345 586**

(52) Cooperative Patent Classification (CPC): (Cont.)

C-Sets
A61M 2230/432, A61M 2230/005

**Description**

TECHNICAL FIELD

The present invention relates to a computer program and a breathing apparatus for enabling non-invasive determination of at least one physiological parameter related to the effective lung volume, the cardiac output, the effective pulmonary blood flow and/or the carbon dioxide content of venous blood of a mechanically ventilated subject.

BACKGROUND

Monitoring physiological parameters such as the effective lung volume (ELV), the effective pulmonary blood flow (EPBF), the cardiac output (Q) and the carbon dioxide content of venous blood is important when the cardiovascular stability and/or the lung function of a subject is potentially threatened, e.g. during surgery or in critically ill patients. For example, it is often desired to monitor one or more of said parameters during ventilatory treatment of a patient.

Most non-invasive respiratory based methods for determination of EPBF or cardiac output are based on some form of the basic physiological principle known as the Fick principle. According to the Fick equation, the cardiac output of a patient may be determined using the following basic relationship:

$$Q = \frac{VCO2}{\left(CvCO2 - CaCO2\right)} \qquad\qquad \text{(eq. 1)}$$

where Q is cardiac output, VCO2 is the volume of carbon dioxide excreted from the body of a patient during respiration (carbon dioxide elimination), CvCO2 is the carbon dioxide concentration in venous blood of the patient, and CaCO2 is the carbon dioxide concentration in arterial blood of the patient.

As well known in the art, EPBF is directly derivable from the cardiac output as:

$$Q \cdot \left(1 - fs\right) = EPBF \qquad\qquad \text{(eq. 2)}$$

Typically, in order to non-invasively determine the cardiac output or EPBF of the patient, a differential form of the carbon dioxide Fick equation is used. Differential Fick techniques are normally based on the premise that cardiac output and EPBF can be estimated based on measurable changes in VCO2, caused by a change in the effective ventilation of the patient. Known ways of changing the effective ventilation of mechanically ventilated patients in order to cause a desired change in VCO2 include changing the degree of rebreathing of expiration gases by means of partial rebreathing through a non-invasive cardiac output (NICO) loop, changing the degree of rebreathing of expiration gases by means of partial rebreathing through an inspiratory line of the ventilator, changing the tidal volume of breaths delivered to the patient, changing the respiratory rate, changing the inspiratory and/or expiratory times, changing the duration of the end-inspiratory pause between inspiration and expiration, and changing the positive end-expiratory pressure (PEEP) applied to the patient.

When the change in effective ventilation is applied, the ventilation of the patient is typically changed from a baseline (normal) level of ventilation to a level of increased or decreased ventilation, depending on the type of change in effective ventilation.

Techniques for non-invasive determination of cardiac output or EPBF often employs a ventilation pattern in which a first phase of baseline ventilation is followed by a second phase of decreased ventilation which is long enough in order for the VCO2 of the patient to reach a new steady state level. The cardiac output of the patient may then be determined based on a comparison between a first steady state level of VCO2 obtained during baseline ventilation and a second steady state level of VCO2 obtained after a certain period of decreased ventilation. Calculating cardiac output or EPBF based on measurements obtained during two different steady state levels of VCO2 is advantageous in that the ELV of the patient need not to be known or taken into account during determination.

Early steady-state techniques suggested said second phase of decreased ventilation to be followed by a recovery phase in which ventilation is allowed to return to normal before again starting to measure respiratory CO2 and flow. This type of ventilation pattern is used e.g. by the so called NICO system which employs a cyclic ventilation pattern comprising a 60 second period of baseline ventilation, a 50 second period of decreased ventilation (rebreathing), and a 70 second recovery period. Consequently, each cycle of the cyclic ventilation pattern lasts for about 3 minutes. Another exemplary technique having a total cycle time of about 3 ½ minutes, with a phase of decreased ventilation lasting for about 30 seconds, is disclosed in Capek, J M, and Roy, RJ, Noninvasive measurement of cardiac output using partial CO2 rebreathing, IEEE Trans. Biomed. Eng. 1988; 35:653-661. Yet another technique employing a cyclic ventilation pattern comprising recovery phases is disclosed in Gama de Abreu, M, et al., Partial carbon dioxide rebreathing: A reliable technique for noninvasive measurement of nonshunted pulmonary capillary blood flow, Crit. Care Med. 1997; 25:675-683. This technique employs a cyclic ventilation pattern having a total cycle time of about 3 minutes, and a period of decreased ventilation (rebreathing) of about 35 seconds.

The disadvantage of the above discussed techniques employing recovery phases between phases of baseline ventilation and phases of changed (e.g. decreased) ventilation is the long response time in determination of cardiac output and EPBF. Having a total cycle time of 3 minutes or more, these techniques must be regarded as intermittent techniques rather than continuous techniques for determination of cardiac output and EPBF. Continuous techniques shortening the response time in cardiac output and EPBF determination is crucial e.g. during mechanical ventilation of critically ill patients.

Therefore, US 7135001 by Orr et al. suggests a cyclic ventilation pattern in which the recovery period during which the patient's respiration is allowed to return to normal before again measuring respiratory CO2 and flow is omitted. The cyclic ventilation pattern proposed by Orr et al. includes two phases: a "normal" (baseline) respiration phase and a phase of in which a change in effective ventilation of the patient is induced (the "change-inducing phase"). These phases are abbreviated in duration relative to the time lengths of the corresponding phases in conventional Fick-based techniques for non-invasive determination of cardiac output or EPBF. The total cycle time of the proposed ventilation pattern may be 2 minutes or less and, preferably, the duration of each phase should be within the interval of approximately eighteen to approximately forty-two seconds. The phases should, however, be long enough in order for VCO2 to reach a substantially steady state in each phase of normal ventilation and each phase of changed ventilation. Thus, Orr et al. discloses a steady-state based technique wherein recovery phases are omitted.

Another technique for non-invasive determination of cardiac output and EPBF employing a cyclic ventilation pattern having relatively short cycles is disclosed in Peyton et al., "Noninvasive, automated and continuous cardiac output monitoring by pulmonary capnodynamics: breath-by-breath comparison with ultrasonic flow probe", Anesthesiology 2006 Jul; 105(1):72-80, which technique is further described in WO 2006/119546 A1. This technique provides for non-invasive, automated and continuous (breath-by breath) determination of cardiac output and is referred to as a capnodynamic technique due to the preferred use of CO2 measurements in the determination of cardiac output. In WO2006/119546, the technique employs a continuous alternating/cyclic alveolar ventilation pattern, with each period of alveolar ventilation at a particular level (hyperventilation or hypoventilation) constituting a half cycle. Preferably, a cycle comprises 6 to 20 breaths, typically 12 breaths; a half cycle being half of this number of breaths. The method employs a "calibration equation" which has to be solved for breaths that occur at periods in the half cycles during which washin or washout of carbon dioxide is minimised, i.e. for breaths occurring when the level of expired CO2 has reached a substantially steady state following a change in effective ventilation. Consequently, Peyton et al. also discloses a steady-state based technique wherein recovery phases are omitted.

Lately, new techniques which do not require the level of expired CO2 to assume a steady state within the alternating phases of increased and decreased ventilation have been proposed. In WO2013/141766 by Emtell and Hallbäck, a non-invasive, capnodynamic method for determination of physiological parameters related to ELV, cardiac output and/or CvCO2 is disclosed. The method is based on a three-dimensional correlation analysis allowing said three parameters to be determined simultaneously on a breath-by-breath basis from expiratory flow and CO2 measurements. The method is independent of the ventilation pattern applied to the subject and requires a change in the level of expired CO2 of about 0,5 percentage unit during the analysed sequence of breaths. To obtain the required change in expired CO2, the method may involve application of a cyclic ventilation pattern to the ventilated patient, comprising alternating phases of hyperventilation (increased ventilation) and hypoventilation (decreased ventilation). For example, each cycle of the cyclic ventilation pattern may comprise a sequence of five hyperventilated breaths followed by a sequence of five hypoventilated breaths.

EP2799008 by Emtell and Hallbäck and seeking priority from WO2013/141766 discloses a non-invasive method for determination of physiological parameters related to ELV, cardiac output and/or CvCO2 employing a cyclic ventilation pattern comprising at least three and no more than five breaths in total. In a preferred embodiment, the cyclic ventilation pattern comprises three breaths of increased ventilation and two breaths of decreased ventilation. The method requires use of algorithms which does not require the level of expired CO2 to reach a steady state level between changes in effective ventilation of the patient since the sequences of increased and decreased ventilation are generally too short for steady state to occur.

Compared to known techniques using longer cyclic ventilation patterns, cyclic ventilation patterns comprising short sequences of increased and decreased ventilation has the advantage of reducing the response time in the determination of the unknown physiological parameters, thus providing for highly responsive monitoring of said physiological parameters. Furthermore, short sequences of increased and decreased ventilation reduce the risk of introducing variations in the CO2 content of venous blood of the patient, which risk is particularly high at high levels of cardiac output. Yet further, short sequences of increased and decreased ventilation reduce the potentially adverse effects on the patient caused by the changes in effective ventilation.

However, a disadvantage of using short sequences of increased and decreased ventilation during determination of cardiac output or other physiological parameters is that the determination cannot be made based on measurements obtained during periods in which the level of expired CO2 assumes a steady state. Therefore, the ELV of the patient cannot be assumed to be constant during measurements and thus needs to be taken into account in the calculations. Involving ELV in the calculation of e.g. cardiac output or EPBF makes calculations more complex. Furthermore, errors in ELV determination inevitably introduce errors in the determination of cardiac output and EPBF.

Consequently, there is a need for a technique allowing physiological parameters such as ELV and EPBF to be determined non-invasively by means of a method providing short response time but yet high accuracy in parameter determination.

The patent application US 2013/345586 A1 (FISHER ET AL, 2013-12-26) is pertinent to understand the background of the invention. This application discloses the non-characterizing section of the appended independent claims.

SUMMARY OF THE DISCLOSURE

It is an object of the invention to enable non-invasive determination of physiological parameters relating to the effective lung volume (ELV), cardiac output, effective pulmonary blood flow (EPBF) or carbon dioxide content of venous blood of a mechanically ventilated subject.

It is a particular object of invention to enable at least one physiological parameter related to the ELV, cardiac output, EPBF and/or the CO2 content of venous blood of a mechanically ventilated subject to be non-invasively determined while at the same time solving or mitigating one of the above discussed shortcomings of prior art.

This and other objects are achieved by a computer program and a breathing apparatus as set forth in the appended claims. The appended claims define the scope of the invention.

According to one aspect of the present disclosure there is provided a method for enabling determination of at least one physiological parameter related to the ELV, cardiac output, EPBF and/or the CO2 content of venous blood of a subject from flow and CO2 measurements obtained during mechanical ventilation of said subject by means of a breathing apparatus. The method comprises the step of ventilating the subject using a ventilation pattern comprising at least one phase of decreased ventilation and at least one phase of increased ventilation, wherein each of the phase of decreased ventilation and the phase of increased ventilation comprises at least two breaths during which a level of CO2 expired by said subject assumes a substantially steady state. At least one, and preferably both, of said phases of decreased and increased ventilation comprises at least a first breath for generating a substantial change in the level of expired CO2 compared to a preceding breath, and at least a second breath being different in duration and/or volume than said first breath, for causing the level of expired CO2 to assume said substantially steady state.

Thus, for each phase of decreased and/or increased ventilation, the proposed method involves the step of changing the effective ventilation of the subject a first time to initiate said phase of decreased or increased ventilation, and at least a second time by changing the duration and/or volume of breaths delivered by the breathing apparatus to actively cause the level of expired CO2 to assume a substantially steady state within said phase of decreased or increased ventilation.

The proposed technique may be referred to as a "forced steady-state technique" as the at least second change in effective ventilation for each phase of decreased and/or increased ventilation serve the purpose of forcing the level of expired CO2 towards a substantially steady state following a first, substantial change in expired CO2. This is very different from steady-state based techniques according to prior art, in which the level of expired CO2 is allowed to gradually and passively reach a new steady state following a fixed and static change in active ventilation of the patient. While known methods switches between phases of decreased and increased ventilation by applying a step change in effective ventilation, causing a gradual change in expired CO2 in breaths following said step change, the proposed method of forced steady state changes the effective ventilation of the subject dynamically in order to achieve a step change or near step change in the level of expired CO2.

An effect of the proposed forced steady-state technique is that the ventilation pattern can be made very short in duration while still providing the advantages associated with longer ventilation patterns allowing the level of expired CO2 to reach a steady state in between changes in effective ventilation.

According to the invention defined in the appended claims, said first change in effective ventilation is effectuated by a change in the duration and/or volume of breaths delivered to the subject. This means that, the duration and/or volume of breaths delivered to the subject is changed a first time to generate said at least first breath causing the substantial change in the level of expired CO2, which at least first breath thus differ in duration and/or volume from a preceding breath, and a second time to generate said at least second breath for causing the level of expired CO2 to assume a substantially steady state level during the phase of decreased or increased ventilation, wherein the second change in duration and/or volume of breaths is different than said first change.

The at least one phase of decreased ventilation is initiated by a first change (decrease) in effective ventilation of the patient, causing a substantial increase in the level of CO2 expired by the patient. This first change in effective ventilation is preferably effectuated by changing (prolonging) the duration of said at least first breath as compared to a preceding breath, which preceding breath is typically the last breath in a preceding phase of increased ventilation. The change in duration of the at least first breath may be effectuated by prolonging an inspiratory pause of said at least first breath, e.g. a pre-inspiratory pause and/or an end-inspiratory pause. Then, within said phase of decreased ventilation, a second change in the effective ventilation of the patient may be made to prevent further increase in the level of expired CO2 and to cause said level of expired CO2 to assume a substantially steady state during at least two breaths of said phase of decreased ventilation, typically during two consecutive breaths, such as a first and a second breath, and/or a second and a third breath, of the phase of decreased ventilation. This second change in effective ventilation is preferably effectuated by changing (shortening) the duration of said at least second breath as compared to said at least first breath, e.g. by shortening a pre-inspiratory pause of the at least second breath.

The at least one phase of increased ventilation is initiated by a first change (increase) in effective ventilation of the patient, causing a substantial decrease in the level of expired CO2. This change in effective ventilation is preferably effectuated by changing (shortening) the duration of said at least first breath and/or by changing (increasing) the volume of said at least first breath as compared to a preceding breath, which preceding breath is typically the last breath in a preceding phase of decreased ventilation. The change in duration of the at least first breath may be effectuated by removing or shortening an inspiratory pause of said at least first breath, e.g. a pre-inspiratory pause of said at least first breath, and the change in volume of the at least first breath may be effectuated by increasing the tidal volume of said at least first breath. Then, within said phase of increased ventilation, a second change in the effective ventilation of the patient may be made to prevent further decrease in the level of expired CO2 and to cause said level of expired CO2 to assume a substantially steady state during at least two breaths of said phase of increased ventilation, typically during two consecutive breaths, such as a first and a second breath, and/or a second and a third breath of the phase of increased ventilation. This second change in effective ventilation is preferably effectuated by changing (increasing) the duration of said at least second breath as compared to said at least first breath, e.g. by prolonging a pre-inspiratory pause of the at least second breath, and/or by changing (decreasing) the volume of said at least second breath as compared to said at least first breath, e.g. by decreasing the tidal volume of the said at least second breath.

The at least first breath in the phase of decreased and/or increased ventilation should cause a substantial change in the level of expired CO2 compared to a preceding breath, which change should be at least 0,3 percentage units and preferably in the range of 0,3-1 percentage unit when measured as fraction of CO2 in expiration gas. Said substantial change in the level of expired CO2 is caused by one single first breath which differs in duration and/or volume from a preceding breath to an extent required to effectuate said substantial change. In the same phase of decreased or increased ventilation, at least a second breath being different in duration and/or volume than said first breath is delivered to the patient for causing level of expired CO2 to assume a substantially steady state during at least two breaths of said phase. Preferably, the at least second breath is one single second breath directly following said first single breath, which second breath is adapted in duration and/or volume to prevent further changes in expired CO2, thereby maintaining the level of expired CO2 during said second breath at or near the level of expired CO2 during said first breath. If the prevailing circumstances do not allow a substantially steady state of expired CO2 to be reached between the first and second breaths, a third breath being different in duration and/or volume from both said first breath and said second breath may be delivered to the patient to cause the level of expired CO2 to assume a substantially steady state between said second breath and said third breath.

The main advantage of the proposed ventilation pattern is that it provides for short response time in non-invasive determination of the physiological parameters while allowing EPBF, cardiac output and/or the CO2 content of venous blood to be determined from two steady states of expired CO2 from which these parameters can be determined independently of the ELV of the patient, or at least from two substantially steady states of expired CO2 in which the ELV of the patient has small impact on EPBF, cardiac output and/or the CO2 content of venous blood. Compared to non-steady-state techniques in which ELV has to be estimated in order to determine the EPBF, the cardiac output and/or the CO2 content of venous blood of the patient, the proposed technique provides for increased accuracy in the determination of EPBF, cardiac output and the CO2 content of venous blood since errors in ELV estimation do not affect the determination of EPBF, cardiac output and/or the CO2 content of venous blood. Furthermore, it enables less complex algorithms to be used for non-invasive determination of EPBF, cardiac output and/or the CO2 content of venous blood and so reduces the demand for computational power. CvCO2 (the CO2 concentration in venous blood) and PvCO2 (the partial pressure of CO2 in venous blood) are non-exclusive examples of parameters relating to the CO2 content of venous blood.

Another advantage of the proposed technique is that it provides for accurate determination of ELV from transient breaths during which changes in ELV are caused mainly by changes in the duration of the breathing cycle ($\Delta t$) or VCO2 rather than changes in EPBF and the CO2 content of venous blood. The at least first breath in the phase of decreased and/or increased ventilation, for generating a substantial change in the level of expired CO2 compared to a preceding breath, together with said preceding breath, form at least two transient breaths from which the ELV of the patient may be advantageously determined.

An advantage of effectuating the change in effective ventilation by changing the duration and/or the volume of delivered breaths is that these type of changes are less prone to adversely affect the average ventilation of the patient over time as compared to other types of changes in effective ventilation, such as changes in effective ventilation caused by partial rebreathing of CO2-containing exhalation gases. Yet another advantage is that existing breathing apparatuses, e.g. most modern ventilators, can be adapted to deliver the proposed ventilation pattern merely by updating the software controlling the operation of the ventilator. No additional hardware or hardware components, e.g. in form of NICO loops or the like, are required.

To effectuate the substantial change in the level of expired CO2 when switching from increased to decreased ventilation, the at least first breath of decreased ventilation may comprise a pre-inspiratory pause which is prolonged compared to any pre-inspiratory pause of said preceding breath, in order to effectuate said substantial change (i.e. increase) in the level of expired CO2. Effectuating the decrease in effective ventilation by prolonging the pre-inspiratory pause has been proved lenient to the perfusion of the patient's lung, and so to provide for reliable determination of EPBF, cardiac output and/or the CO2 content of venous blood from breaths of steady state following said change in effective ventilation. Preferably, the at least second breath in the phase of decreased ventilation comprises a pre-inspiratory pause that is shorter than the pre-inspiratory pause of said at least first breath in the phase of decreased ventilation. Shortening or removing the pre-inspiratory pause of the at least second breath of decreased ventilation has been proved to provide a simple and efficient way of forcing the level of CO2 to assume a steady state after the initial increase in expired CO2 caused by the prolonged pre-inspiratory pause of the at least first breath of decreased ventilation.

Alternatively, to effectuate the substantial change in the level of expired CO2 when switching from increased to decreased ventilation, the at least first breath of decreased ventilation may comprise an end-inspiratory pause which is prolonged compared to any end-inspiratory pause of the preceding breath. This is in alternative to the above mentioned prolongation of the pre-inspiratory pause of the at least first breath of decreased ventilation. Prolongation of the end-inspiratory pause has been found advantageous compared to prolongation of the pre-inspiratory pause in that determination of ELV becomes more robust and reliable when determined from transient breaths where the transient change in expired CO2 is caused by prolongation of the end-inspiratory pause instead of prolongation of the pre-inspiratory pause. Furthermore, the effect of the change in duration of the end-inspiratory pause is shown in the expiration following immediately thereafter, which is advantageous since the pulmonary dynamics following the effective change in ventilation becomes easier to model, thus providing for more reliable determination of ELV. The effect of a change in duration of a pre-inspiratory pause on the other hand is not shown until the expiration following the inspiration immediately following said pre-inspiratory pause. This makes the pulmonary dynamics more difficult to analyse and the model of the pulmonary dynamics more sensitive to deficiencies in the mathematical description of the gas exchange in the lung of the subject. Preferably, to force the level of expired CO2 to assume a substantially steady state following the substantial increase caused by the prolongation of the end-inspiratory pause, the at least second breath in the phase of decreased ventilation has no end-inspiratory pause but a pre-inspiratory pause which is prolonged compared to the pre-inspiratory pause (if any) of said at least first breath of decreased ventilation.

To effectuate the substantial change in the level of expired CO2 when switching from decreased to increased ventilation, the at least first breath of increased ventilation may comprise an increased tidal volume compared to the tidal volume of a preceding breath, in order to effectuate said substantial change (i.e. decrease) in the level of expired CO2. The increase in tidal volume causes a forced wash-out of CO2 from the patient's lung, effectively increasing the effective ventilation of the patient. Preferably, the at least second breath in the phase of increased ventilation is a breath of decreased tidal volume as compared to said at least first breath in the phase of increased ventilation. Decreasing the tidal volume of the at least second breath of increased ventilation, which may be achieved by removing the temporary increase in tidal volume of the at least first breath of increased ventilation, has been proved to be a simple and efficient way of forcing the level of CO2 to assume a steady state after the initial decrease in expired CO2 caused by the increased tidal volume of the at least first breath of increased ventilation.

Instead of or in addition to an increased tidal volume, the at least first breath of increased ventilation may comprise a pre-inspiratory pause which is shortened compared to any pre-inspiratory pause of said preceding breath in order to effectuate said substantial change (i.e. increase) in the level of expired CO2. Typically, in this scenario, said at least first breath comprises no or only a short pre-inspiratory pause. Shortening of the pre-inspiratory paus also causes a forced wash-out of CO2 from the patient's lung, effectively increasing the effective ventilation of the patient while leaving the perfusion of the patient's lung substantially unaffected. To force the level of expired CO2 to assume a substantially steady state following the substantial decrease caused by the shortening of the pre-inspiratory pause, the at least second breath of increased ventilation following said at least first breath of increased ventilation may comprise a pre-inspiratory pause that is prolonged compared to the pre-inspiratory pause (if any) of said at least first breath of increased ventilation. Consequently, the proposed technique of forced steady state may be implemented also using a ventilation pattern comprising breaths of equal tidal volume, differing from each other only in duration.

Typically, the proposed ventilation pattern is a cyclic ventilation pattern comprising alternating phases of decreased and increased ventilation. In theory, a cyclic ventilation pattern comprising no more than two breaths of decreased ventilation and two breaths of increased ventilation could be used to obtain the above mentioned advantages since ELV could be reliably determined from the transient breaths of different types while EPBF, cardiac output and/or the CO2 content of venous blood could be reliably determined from two breaths of decreased ventilation and two breaths of increased ventilation, respectively, during which the level of expired CO2 remains substantially the same. In practice, however, cycles of more than four breaths may sometimes be desirable. Thus, each cycle of the cyclic ventilation pattern may comprise four or more breaths, at least two being breaths of decreased ventilation and at least two being breaths of increased ventilation. Preferably, the cyclic ventilation pattern comprises four to ten breaths, more preferably five to eight breaths, and most preferably six to seven breaths of which three are breaths of decreased ventilation and three or four are breaths of increased ventilation. The technique of forced steady state can be applied within any or both of said phases of decreased and increased ventilation. If applied only within one of the phases, the other phase must typically comprise a larger number of breaths, typically six or more, in order for the level of expired CO2 to passively reach a substantially steady state within said phase.

Preferably, the breaths of increased ventilation are hyperventilated breaths and the breaths of decreased ventilation are hypoventilated breaths. Thereby, the total ventilation over time can be made to correspond to a desired optimal ventilation of the subject. In this regard it should be emphasized that phases of increased and decreased ventilation should not be construed as being limited to phases of ventilation that are increased and decreased in relation to baseline (normal) ventilation. Instead, it should be understood that in the context of this application, a phase of decreased ventilation is a phase in which ventilation is decreased compared to a phase of increased ventilation, and vice versa. Thus, it should be realized that embodiments wherein the level of ventilation in the phase of increased ventilation or the level of ventilation in the phase of decreased ventilation corresponds to a baseline level of ventilation are also contemplated by the present invention.

In view of the above, it should be appreciated that the duration and/or the volume of breaths delivered by the breathing apparatus may be dynamically changed during the phases of decreased and/or increased ventilation in order to deliver a cyclic ventilation pattern causing the level of expired CO2 to vary essentially in accordance with a trapezoidal (near square) waveform, the upper plateaus of which correspond to phases of decreased ventilation and the lower plateaus of which correspond to phases of increased ventilation. This allows EPBF, cardiac output and/or the CO2 content of venous blood of the patient to be reliably determined from breaths of said upper or lower plateaus, and the ELV of the patient to be reliably determined from transient breaths during which the level of expired CO2 goes from a lower plateau to an upper plateau, or vice versa.

The method may further comprise a step of determining the at least one physiological parameter relating to ELV, EPBF, cardiac output and/or the CO2 content of venous blood of the ventilated subject. To this end, the method may comprise one or both of the steps of:

i) determining the EPBF, cardiac output and/or the CO2 content of venous blood of the ventilated subject from an analysed sequence of breath comprising at least two breaths of a first and substantially steady state level of expired CO2 and at least two breaths of a second substantially steady state level of expired CO2, being different than said first substantially steady state level, and
ii) determining the ELV of the ventilated subject from an analysed sequence of breath comprising at least two transient breaths, meaning that the levels of expired CO2 differ substantially between said breaths.

Preferably, the method comprises both step i) and step ii), meaning that both ELV and at least one of EPBF, cardiac output and the CO2 content of venous blood are determined from an analysed sequence of breath during which the subject is ventilated using the proposed ventilation pattern.

Although the proposed technique of forced steady state strives at causing the level of expired CO2 to assume a steady state within the phase of decreased and/or increased ventilation, it may not be possible to reach a perfectly steady state in all circumstances. Therefore, the determination of the at least one physiological parameter is preferably made using an algorithm which does not require the level of expired CO2 to assume a steady state within the phases of decreased and increased ventilation, meaning that the algorithm should be able to derive a value of at least one of said physiological parameters even if steady state is not reached. Preferably, the method employs an algorithm which is capable of deriving a value of EPBF, cardiac output and/or the CO2 content of venous blood even if steady state is not reached within the phases of decreased and increased ventilation. Of course, this is advantageous should the proposed ventilation pattern fail to cause a steady state to be reached during said analysed sequence of breaths.

Step i) typically involves determination of EPBF, cardiac output and/or the CO2 content of venous blood from at least two breaths of substantially steady state within a phase of decreased ventilation and at least two breaths of substantially steady state within a phase of increased ventilation. Breaths of substantially steady state should herein be construed as breaths, preferably but not necessarily consecutive breaths, between which the levels of expired CO2, when measured as fraction of CO2 in expiration gas (e.g. FetCO2), deviate from each other by no more than 0,1 percentage units, preferably no more than 0,05 percentage units, and most preferably no more than 0,025 percentage units.

Step ii) may involve determination of ELV from at least two transient breaths between a phase of increased ventilation and a phase of decreased ventilation, or vice versa. In some embodiments it may involve determination of ELV from at least a first sequence (two or more) of transient breaths between a phase of increased ventilation and a phase of decreased ventilation, and a second sequence (two or more) of transient breaths between a phase of decreased ventilation and a phase of increased ventilation. Preferably, said transient breaths include said at least first breath for generating a substantial change in the level of expired CO2 compared to a preceding breath, and said preceding breath. The substantial change in the level of expired CO2, when measured as fraction of CO2 in expiration gas (e.g. FetCO2), should be at least 0,3 percentage units, preferably at least 0,4 percentage units, more preferably at least 0,5 percentage units, and most preferably in the range of 0,5-1 percentage unit. For example, ELV may be determined from two transient breaths between which the level of expired CO2 changes from about 4,8% to about 5,5%. Accordingly, transient breaths should herein be construed as a sequence of two or more breaths between which the total change in the level of expired CO2, when measured as fraction of CO2 in expiration gas (e.g. as FetCO2), is at least 0,3 percentage units, preferably at least 0,4 percentage units, more preferably at least 0,5 percentage units and most preferably between 0,5-1 percentage unit. Consequently, the transient breaths constitute a sequence of two or more breaths wherein the level of expired CO2 in the first breath of said sequence differs from the level of expired CO2 in the last breath of said sequence by at least 0,3 percentage units when measured as fraction of CO2 in expiration gas (e.g. as FetCO2).

Preferably, the method involves determination of both ELV and at least one of EPBF, cardiac output and the CO2 content of venous blood. WO2013/141766 discloses a non-invasive and continuous method for simultaneous determination of ELV, cardiac output and CvCO2 (i.e. the CO2 concentration of venous blood) which may be advantageously used together with the proposed ventilation pattern of forced steady state in order to more accurately determine ELV, cardiac output, the CO2 content of venous blood and EPBF (directly derivable from the cardiac output).

The advantage of using the proposed ventilation pattern of forced steady state instead of the ventilation pattern employed in WO2013/141766 (which is generally too short to reach steady state) is that the substantially steady state of expired CO2 obtained within the phases of decreased and increased ventilation provides for higher accuracy in the determination of cardiac output, EPBF and the CO2 content of venous blood, while the distinct transients between phases of decreased and increased ventilation provide for higher accuracy in ELV determination.

Thus, in accordance with the teachings of WO2013/141766, the step of determining the at least one parameter related to ELV, cardiac output, EPBF and/or the CO2 content of venous blood of the ventilated subject may comprise the steps of:

- determining, for each breath in an analysed sequence of breaths, a first parameter related to the fraction of alveolar CO2 of the subject, a second parameter related to the CO2 content of the arterial blood of the subject, and a third parameter related to CO2 elimination of the subject, based on measurements of at least an expiratory flow of expiration gas exhaled by the subject, and a CO2 content of at least the expiration gas exhaled by the subject, and
- determining said at least one physiological parameter based on the correlation of the first, second and third parameters in said sequence of analysed breaths. Preferably but not necessarily, the number of breaths in said sequence of analysed breaths corresponds to the number of breaths in each cycle of the cyclic ventilation pattern.

According to another aspect of the present disclosure there is provided a breathing apparatus, such as a ventilator or an anaesthesia apparatus, capable of carrying out the above described method of enabling non-invasive determination of at least one physiological parameter related to the ELV, cardiac output, EPBF and/or the CO2 content of venous blood of a subject from flow and CO2 measurements obtained during mechanical ventilation of said subject.

To this end, there is provided a breathing apparatus for providing mechanical ventilation to a subject, comprising a control unit configured to control the operation of the breathing apparatus and thereby the ventilation of the mechanically ventilated subject. The control unit is configured to control the operation of the breathing apparatus such that the subject is ventilated using a ventilation pattern comprising at least one phase of decreased ventilation and at least one phase of increased ventilation, wherein each of the phase of decreased ventilation and the phase of increased ventilation comprises at least two breaths during which a level of CO2 expired by said subject assumes a substantially steady state. The control unit is configured to control the operation of the breathing apparatus such that at least one and preferably both of said phases of decreased and increased ventilation comprises at least a first breath for generating a substantial change in the level of expired CO2 compared to a preceding breath, and at least a second breath being different in duration and/or volume than said first breath, for causing the level of expired CO2 to assume said substantially steady state.

Typically, the breathing apparatus comprises a pneumatic unit for delivery of pressurised breathing gas to the ventilated subject, the control unit being configured to control the pneumatic unit to deliver breaths of breathing gas to the subject in accordance with said ventilation pattern.

The control unit may be configured to cause the breathing apparatus to deliver any of the above discussed ventilation patterns in which ventilation switches from a phase of increased ventilation to a phase of decreased ventilation, and/or vice versa, by first causing a substantial change in the level of CO2 expired by the subject, and then forcing the level of expired CO2 to a substantially steady state.

This may be achieved by the control unit by varying the pre-inspiratory pause, the end-inspiratory pause and/or the tidal volume of the breaths delivered by the breathing apparatus, as described above.

Preferably, the breathing apparatus is further configured to determine the at least one physiological parameter relating to ELV, EPBF, cardiac output and/or the CO2 content of venous blood of the ventilated subject. To this end, the breathing apparatus may comprise a flow sensor for measuring at least an expiratory flow of expiration gas exhaled by the subject, and a CO2 sensor for measuring the CO2 content of at least the expiration gas exhaled by the subject. The control unit may be configured to determine said at least one physiological parameter from flow and CO2 measurements obtained by said sensors during an analysed sequence of breaths during which the subject is ventilated using said ventilation pattern.

Preferably, the control unit is configured to determine said at least one physiological parameter using an algorithm which does not require the level of expired CO2 to reach a steady state during the analysed sequence of breaths.

Preferably, the control unit is configured to determine both ELV and at least one of EPBF, cardiac output and the CO2 content of venous blood from the flow and CO2 measurements obtained during said analysed sequence of breaths.

In an exemplary embodiment, the breathing apparatus comprises a flow sensor and a CO2 sensor arranged in or close to a Y-piece connecting an inspiratory branch and an expiratory branch of the breathing apparatus with the patient. The flow sensor may advantageously be configured to measure both the inspiratory and expiratory flow to and from the patient continuously to obtain a continuous flow curve representing the flow of gas into and out of the airways of the patient over time. Likewise, the CO2 sensor may be configured to measure the CO2 content in the inspiration and expiration gas continuously to obtain a continuous CO2 fraction curve representing the carbon dioxide content inhaled and exhaled by the patient over time. The control unit of the breathing apparatus may be configured to use the flow and carbon dioxide content measurements to determine a first, second and third parameter related to the fraction of alveolar CO2, the CO2 content of arterial blood and the CO2 elimination of the subject, respectively, for each breath in the analysed sequence of breaths, and to determine the at least one physiological parameter related to the ELV, cardiac output, EPBF and/or the CO2 content of venous blood of the ventilated subject based on the correlation of said first, second and third parameters in the analysed sequence of breaths.

The logic required to enable the breathing apparatus to carry out the method is preferably implemented by means of software. Thus, according to another aspect of the invention, there is provided a computer program for enabling determination of at least one physiological parameter related to the ELV, cardiac output, EPBF and/or the CO2 content of venous blood of a subject from flow and CO2 measurements obtained during mechanical ventilation of said subject by means of a breathing apparatus. The computer program comprises computer-readable code which, when executed by a processor of the breathing apparatus, e.g. a processor of said control unit, causes the breathing apparatus to ventilate the subject using a ventilation pattern comprising at least one phase of decreased ventilation and at least one phase of increased ventilation, wherein each of the phase of decreased ventilation and the phase of increased ventilation comprises at least two breaths during which a level of CO2 expired by said subject assumes a substantially steady state. The computer-readable code, when executed by said processing unit, causes at least one and preferably both of said phases of decreased and increased ventilation to comprise at least a first breath for generating a substantial change in the level of expired CO2 compared to a preceding breath, and at least a second breath being different in duration and/or volume than said first breath, for causing the level of expired CO2 to assume said substantially steady state.

An advantage of the present invention is that installation of such a computer program on existing breathing apparatuses would allow existing breathing apparatuses to carry out the inventive method.

The computer program may further comprise code segments causing the breathing apparatus to carry out any of the method steps discussed above.

More advantageous aspects of the inventive method, breathing apparatus and computer program will be described in the detailed description of embodiments following hereinafter.

BRIEF DESCRIPTION OF THE DRAWINGS

The present invention will become more fully understood from the detailed description provided hereinafter and the accompanying drawings which are given by way of illustration only. In the different drawings, same reference numerals correspond to the same element.

Fig. 1 illustrates a breathing apparatus according to an exemplary embodiment of the invention;

Figs. 2A-2E illustrate a ventilation pattern according to an exemplary embodiment of the invention;

Figs. 3A-3D illustrate a ventilation pattern according to another exemplary embodiment of the invention;

Figs. 4A-4B illustrate a ventilation pattern according to another exemplary embodiment of the invention, and

Figs. 5A-5E illustrate a ventilation pattern according to yet another exemplary embodiment of the invention.

DETAILED DESCRIPTION

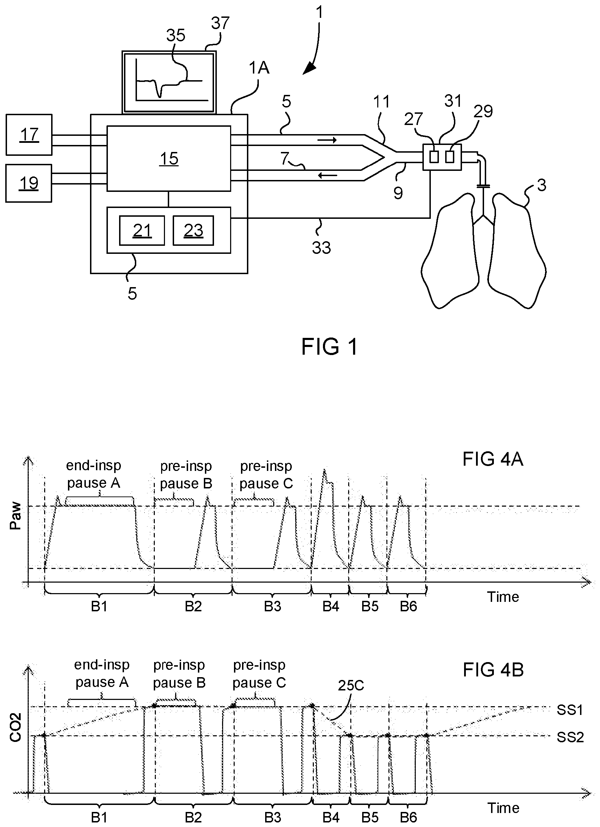

Fig. 1 illustrates a breathing apparatus 1 for enabling continuous and non-invasive determination of one or more physiological parameters related to the effective lung volume (ELV), the cardiac output, the effective pulmonary blood flow (EPBF) and/or the carbon dioxide content of venous blood of a subject 3, according to an exemplary embodiment of the invention.

In this embodiment, the breathing apparatus 1 is a ventilator for providing ventilatory treatment to a subject 3 (herein sometimes referred to as the patient). The ventilator is connected to the patient 3 via an inspiratory line 5 for supplying breathing gas to the patient 3, and an expiratory line 7 for conveying expiration gas away from the patient 3. The inspiratory line 5 and the expiratory line 7 are connected to a common line 9, via a so called Y-piece 11, which common line is connected to the patient 3 via a patient connector, such as an endotracheal tube.

The breathing apparatus 1 further comprises a control unit 13 for controlling the ventilation of the patient 3 based on preset parameters and/or measurements obtained by various sensors of the breathing apparatus. The control unit 13 controls the ventilation of the patient 3 by controlling a pneumatic unit 15 of the breathing apparatus 1, which pneumatic unit 15 is connected at one hand to one or more gas sources 17, 19 and at the other hand to the inspiratory line 5 for regulating a flow and/or pressure of breathing gas delivered to the patient 3. To this end, the pneumatic unit 15 may comprise various gas mixing and regulating means, such as mixing chambers, controllable gas mixing valves and one or more controllable inspiration valves.

The control unit 5 comprises a processing unit 21 and a non-volatile memory 23 storing a computer program which, when executed by the processing unit 21, causes the control unit to control the ventilation of the patient 3 as described hereinafter. Unless stated otherwise, actions and method steps described hereinafter are performed by, or caused by, the control unit 21 upon execution of different code segments of the computer program stored in the memory 23.

The control unit 5 is configured to enable accurate, continuous and non-invasive determination of one or more physiological parameters related to the ELV, the cardiac output, the EPBF and/or the CO2 content of venous blood of the patient 3 by causing the breathing apparatus 1 to ventilate the patient 3 using a ventilation pattern allowing one or more of the cardiac output, the EPBF and/or the CO2 content of venous blood of the patient 3 to be determined substantially independently of ELV, and allowing ELV to be determined from transient breaths during which changes in ELV are caused mainly by changes in the duration of the breathing cycle ($\Delta t$) or VCO2 rather than changes in EPBF and the CO2 content of venous blood.

The ventilation pattern is a cyclic ventilation pattern comprising alternating phases of decreased and increased ventilation. Each phase of decreased ventilation comprises at least a first breath for generating a substantial change in the level of CO2 expired by the patient 3, and at least a second breath, following said at least first breath, for causing said level of expired CO2 to assume a substantially steady state level within the phase of decreased ventilation, i.e. during at least two breaths in said phase of decreased ventilation. Each phase of increased ventilation comprises at least a first breath for generating a substantial and opposite change in the level of CO2 expired by the patient 3, and at least a second breath, following said at least first breath, for causing the level of expired CO2 to assume a new substantially steady state level within the phase of decreased ventilation (i.e. during at least two breaths in the phase of decreased ventilation). The control unit 5 is configured to cause the level of expired CO2 to assume said steady states in the phases of decreased and increased ventilation by changing the duration and/or volume of said at least one second breath with respect to the duration and/or volume of said at least one first breath. Also, the control unit 5 is typically configured to cause said substantial change in the level of expired CO2 by changing the duration and/or volume of said at least one first breath with respect to a breath directly preceding said at least one first breath.

That the level of expired CO2 assumes a substantially steady state during at least two breaths herein means that a measure of expired CO2 obtained during a first breath is substantially equal to a corresponding measure of expired CO2 obtained during a second breath. Said measure of expired CO2 may be any measure indicative of alveolar CO2 of the ventilated patient 3, e.g. a measure of the fraction of alveolar CO2 (FACO2) or a measure of the partial pressure of alveolar CO2 (PACO2), including but not limited to end-tidal fraction of alveolar CO2 (FetCO2) and end-tidal partial pressure of CO2 (PetCO2).

The at least two breaths of the same phase of decreased or increased ventilation during which the level of expired CO2 assumes a substantially steady state will hereinafter be referred to as breaths of steady state. Preferably but not necessarily, the at least two breaths of steady state in the phase of decreased and increased ventilation, respectively, are two or more consecutive breaths within said phase.

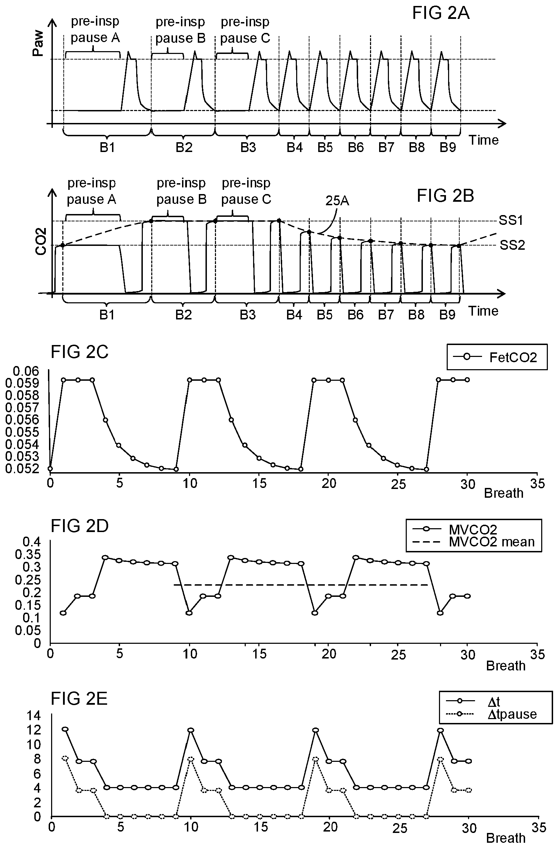

Figs. 2-5 illustrate some examples of cyclic ventilation patterns which, in accordance with the principles described above, may be applied to the patient 3 by the breathing apparatus 1 in order to enable non-invasive determination of one or more of said physiological parameters from flow and CO2 measurements.

Figs. 2A-2E illustrate a ventilation pattern caused by applying the technique of forced steady state only within phases of decreased ventilation while, in phases of increased ventilation, the level of expired CO2 is allowed to passively and gradually reach a substantially steady state. Each cycle of the illustrated ventilation pattern comprises a total of nine breaths B1-B9.

With reference now made to Fig. 2A illustrating the airway pressure of the ventilated patient, e.g. as measured by a pressure sensor (not shown) located in or close to the Y-piece 11 of the breathing apparatus in Fig. 1, a phase of decreased ventilation is initiated by delivering a first breath B1 having a pre-inspiratory pause A which is prolonged compared to any pre-inspiratory pause of a preceding breath (not shown), which preceding breath is the last breath of a preceding phase of increased ventilation.

In Fig. 2B, the solid curve illustrates the variation in CO2 level over time, as measured by a sensor for measuring CO2 content of inspiration and expiration gases inhaled and exhaled by patient being ventilated with the ventilation pattern in Fig. 2A, such as a CO2 sensor 29 of the breathing apparatus 1 in Fig. 1, as will be described in more detail below. The CO2 level may for example be measured as the fraction of CO2 (FCO2) in the gas measured upon during expiration, corresponding to the alveolar fraction of CO2 (FACO2) of the ventilated patient 3. The data points connected by the dashed line 25A represent an end-tidal level of CO2 expired in each breath, in this case corresponding to the end-tidal fraction of CO2 (FetCO2) of each breath. Accordingly, the dashed line 25A illustrates a variation in end-tidal level of expired CO2 over time.

As shown in Fig. 2B, said first breath B1 causes a substantial change in the end-tidal level of CO2 expired by the patient. In a second breath B2, immediately following said first breath, the pre-inspiratory pause B is shortened compared to the pre-inspiratory pause A of the first breath. This shortening of the pre-inspiratory pause prevents further increase in the end-tidal level of expired CO2 and causes the end-tidal level of CO2 expired during said second breath B2 to substantially correspond to the end-tidal level of CO2 expired during the first breath B1. Consequently, the end-tidal level of expired CO2 assumes a substantially steady state, denoted SS1, during said first and second breaths. In this exemplary ventilation pattern, a third breath B3 having a pre-inspiratory pause C corresponding in duration to the pre-inspiratory pause B of the second breath is delivered to the patient following said second breath. This third breath causes the end-tidal level of expired CO2 to remain substantially constant at said level SS1 of steady state. Thus, in this example, the first, second and third breaths in the phase of decreased ventilation are all breaths of steady state as the level of expired CO2 remains substantially constant during said breaths.

After the third breath, a phase of increased ventilation is initiated by delivering a fourth breath B4 having no or only a very short pre-inspiratory pause compared to the preceding breath B3. As mentioned above, the proposed technique of forced steady state is not applied within phases of increased ventilation in this embodiment. This means that after delivery of the fourth breath B4 initiating the phase of increased ventilation, no further changes in effective ventilation are made within said phase of increased ventilation. As illustrated in Fig. 2B, this causes the end-tidal level of expired CO2 to gradually approach a new and lower steady state level SS2. This exemplary ventilation pattern comprises six identical breaths B4-B9 of increased ventilation, which is often sufficient for the end-tidal level of expired CO2 to passively reach a new level of substantially steady state.

Figs. 2C-2E illustrate simulation data from a simulation in which a patient is ventilated by means of a breathing apparatus corresponding to the breathing apparatus 1 in Fig. 1, using a cyclic ventilation pattern in which each cycle corresponds to the ventilation pattern illustrated in Fig. 2A. The simulation data is obtained during a sequence of 30 breaths.

Fig. 2C illustrates the end-tidal fraction of CO2 (FetCO2) for each breath in said sequence of breaths, as measured e.g. by said CO2 sensor 29 of the breathing apparatus 1. As expected, the FetCO2 curve in Fig. 2C resembles the dashed curve 25A illustrating the end-tidal level of expired CO2 in Fig. 2A.

Fig. 2D illustrates the minute elimination (MVCO2) and mean minute elimination (MVCO2 mean) of CO2 of the patient for the same sequence of breaths. The ventilation pattern is preferably adapted such that the mean minute elimination of CO2 is substantially constant. To this end, the breaths of increased ventilation are preferably hyperventilated breaths and the breaths of decreased ventilation are preferably hypoventilated breaths.

Fig. 2E visualizes the changes in effective ventilation of the proposed ventilation pattern and indicates the duration in seconds, $\Delta t$, of each breath in the sequence of breaths, and the duration in seconds, $\Delta t_{pause}$, of the pre-inspiratory pause of each breath in the sequence of breaths. In this exemplary embodiment, the first breath of decreased ventilation comprises a pre-inspiratory pause of approximately eight seconds, the second and third breath of decreased ventilation comprise a pre-inspiratory pause of approximately four seconds, whereas the fourth to ninth breath in each cycle of the cyclic ventilation pattern, corresponding to breaths of increased ventilation, comprises no or only a very short pre-inspiratory pause. The total duration of each cycle of the cyclic ventilation pattern is 52 seconds.

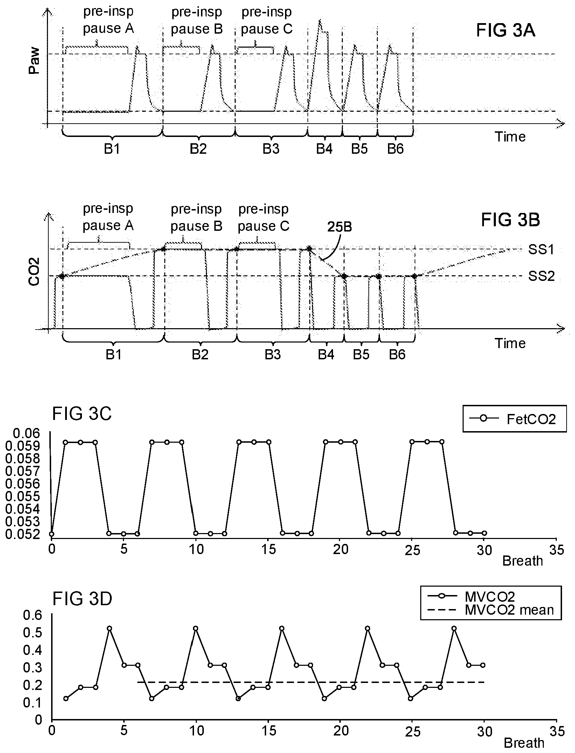

Figs. 3A-3B illustrate an embodiment of a ventilation pattern caused by applying the technique of forced steady state both in phases of decreased ventilation and phases of increased ventilation. In this embodiment, each cycle of the ventilation pattern comprises a total of six breaths B1-B6, three of which are breaths of decreased ventilation and three of which are breaths of increased ventilation. The total duration of each cycle of the cyclic ventilation pattern is 40 seconds.

With reference now made to Fig. 3A, the first three breaths B1-B3 in the phase of decreased ventilation are identical to the first three breaths B1-B3 in Fig. 2A, thus causing a substantial increase in the level of expired CO2 to a first substantially steady state level SS1. In this embodiment, the fourth breath B4 initiating the phase of increased ventilation differs from the preceding breath B3 in both duration and volume. Besides the pre-inspiratory pause of the fourth breath B4 being removed or at least substantially shortened with respect to the pre-inspiratory pause of the preceding breath B3, the tidal volume of the fourth breath B4 is increased compared to the tidal volume of said preceding breath B3. This effectively causes wash-out of CO2 from the lungs of the patient, causing a substantial decrease in the level of CO2 expired by the patient, as shown in Fig. 3B. The first breath of increased ventilation B4 is adapted in duration and volume so as to bring the level of expired CO2 to a level at which it can be maintained for at least one more breath in the same phase of increased ventilation, which level corresponds to the second substantially steady state level SS2.

Preferably, the first breath of increased ventilation B4 should be adapted in duration and/or volume to fully compensate for the substantial increase in the level of expired CO2 caused by the first breath B1 in the phase of decreased ventilation, meaning that said first breath of increased ventilation B4 should bring the level of expired CO2 back to the level of expired CO2 prior to delivery of said first breath of decreased ventilation B1. To maintain the level of expired CO2 at said second substantially steady state level SS2, the fifth breath B5 differ from said fourth breath B4 in duration and/or volume so as to prevent further decrease in the level of expired CO2, and to cause the level of CO2 expired during said fifth breath B5 to substantially correspond to the level of CO2 expired during the preceding fourth breath B4. In this embodiment, the fifth breath B5 differ from the fourth breath B4 in that the temporary change in tidal volume is removed, meaning that the tidal volume of the fifth breath B5 is set to a value substantially corresponding to the tidal volume of the breaths preceding said fourth breath B4. The change in duration of the pre-inspiratory pause of the fourth breath B4 is maintained also for the fifth breath B5, meaning that the fifth breath contains no or only a short pre-inspiratory pause substantially corresponding to the pre-inspiratory pause of the fourth breath B4. After said fifth breath, a sixth breath B6 being identical in duration and volume to the fifth breath is delivered to the patient. This sixth breath causes the level of expired CO2 to remain substantially constant and equal to the second level SS2 of substantially steady state. Thus, in this exemplary ventilation pattern, all breaths of decreased ventilation B1-B3 and all breaths of decreased ventilation B4-B6 are breaths of steady state as the level of expired CO2 remains substantially constant during said breaths.

Figs. 3C-3D illustrate simulation data from a simulation in which a patient is ventilated by means of a breathing apparatus corresponding to the breathing apparatus 1 in Fig. 1, using a cyclic ventilation pattern in which each cycle corresponds to the ventilation pattern illustrated in Fig. 3A. The simulation data is obtained during a sequence of 30 breaths.

Fig. 3C illustrates FetCO2 for each breath in said sequence of breaths, as measured e.g. by said CO2 sensor 29 of the breathing apparatus 1. As expected, the FetCO2 curve in Fig. 3C resembles the dashed curve 25B illustrating variations in the end-tidal levels of CO2 in Fig. 3B.

Fig. 3D illustrates the minute elimination (MVCO2) and mean minute elimination (MVCO2 mean) of CO2 of the patient for the same sequence of breaths. In this embodiment too, the breaths of increased ventilation are hyperventilated breaths and the breaths of decreased ventilation are hypoventilated breaths, which prevents drifting of the mean minute elimination of CO2 and so keeps said mean minute elimination of CO2 substantially constant during ventilation with the proposed ventilation pattern.

In the exemplary ventilation pattern illustrated in Figs. 3A-3D, the tidal volume of the fourth breath B4 may be increased by approximately 55% compared to the tidal volume of the preceding breath B3. According to another exemplary ventilation pattern (not illustrated), the substantial decrease in the level of expired CO2 from the first substantially steady state level SS1 to the second substantially steady state level SS2 may be caused by two consecutive breaths of increased tidal volume, i.e. by two breaths both having a tidal volume that is larger than the breaths preceding said two breaths of increased tidal volume. In one embodiment, said two breaths of increased tidal volume have no pre-inspiratory pauses and tidal volumes that are substantially equal to each other and increased by approximately 19% compared to the tidal volumes of the breaths preceding said two breaths of increased tidal volume. In one embodiment, the fourth breath B4 in Fig. 3A is replaced by said two consecutive breaths of increased tidal volume, resulting in a ventilation pattern comprising seven breaths of which three are breaths of decreased ventilation and four are breaths of increased ventilation, wherein the first breath 1B of decreased ventilation alone is adapted to cause the substantial increase in the level of expired CO2 in the transition between phases of increased ventilation and phases of decreased ventilation, and wherein said two consecutive breaths of increased tidal volume are adapted to cause the substantial decrease in the level of expired CO2 in the transition between phases of decreased ventilation and phases of increased ventilation.

Figs. 4A-4B illustrate another exemplary ventilation pattern in which the proposed technique of forced steady state is applied both in phases of decreased ventilation and phases of increased ventilation. In this embodiment, each cycle of the ventilation pattern comprises a total of six breaths B1-B6, three of which are breaths of decreased ventilation and three of which are breaths of increased ventilation.

With reference now made to Fig. 4A illustrating the airway pressure of the ventilated patient, a phase of decreased ventilation is initiated by delivering a first breath B1 having an end-inspiratory pause A (sometimes referred to as post-inspiratory pause) which is prolonged compared to any end-inspiratory pause of a preceding breath (not shown), which preceding breath is the last breath of a preceding phase of increased ventilation.

As shown in Fig. 4B, said first breath B1 causes a substantial change (increase) in the level of $CO_2$ expired by the patient. In a second breath B2, immediately following said first breath, the end-inspiratory pause is removed or substantially shortened compared to the end-inspiratory pause A of the first breath B1, and a pre-inspiratory pause B is added or substantially prolonged compared to any pre-inspiratory pause of the first breath B1. The removal or shortening of the end-inspiratory pause in the second breath B2 prevents further increase in the level of expired $CO_2$ and causes the level of $CO_2$ expired during said second breath B2 to substantially correspond to the level of $CO_2$ expired during the first breath B1. Consequently, the level of expired $CO_2$ assumes a substantially steady state SS1 during said first and second breaths. In this exemplary ventilation pattern, a third breath B3 having a pre-inspiratory pause C corresponding in duration to the pre-inspiratory pause B of the second breath B2 is delivered to the patient following said second breath. This third breath B3 causes the level of expired $CO_2$ to remain substantially constant at said steady state level SS1. Thus, in this example too, the first, second and third breaths in the phase of decreased ventilation are all breaths of steady state as the level of expired $CO_2$ remains substantially constant during said breaths.

The fourth to sixth breath B4-B6 are identical to the fourth to six breaths B4-B6 in the ventilation pattern illustrated in Fig.3A. This means that a fourth breath B4 differing from the preceding third breath B3 in both duration (no or substantially shortened pre-inspiratory pause) and volume (increased tidal volume) is delivered to the patient to initiate the phase of increased ventilation through forced wash-out of $CO_2$ from the lungs of the patient, abruptly bringing the level of expired $CO_2$ from the first substantially steady state level SS1 to a new substantially lower level SS2. Said fourth breath B4 is immediately followed by a fifth breath B5 differing from said fourth breath B4 in that the temporary change in tidal volume is removed to prevent further decrease in the expired level of $CO_2$ and force the level of $CO_2$ expired during the fifth breath B5 to remain at or near said new and lower level SS2, which level thus constitutes a substantially steady state level of $CO_2$ expired during the fourth and fifth breath. The sixth breath B6 is identical to the fifth breath B5 and serves to maintain the level of $CO_2$ expired during the sixth breath B6 at said second and substantially steady state level SS2.

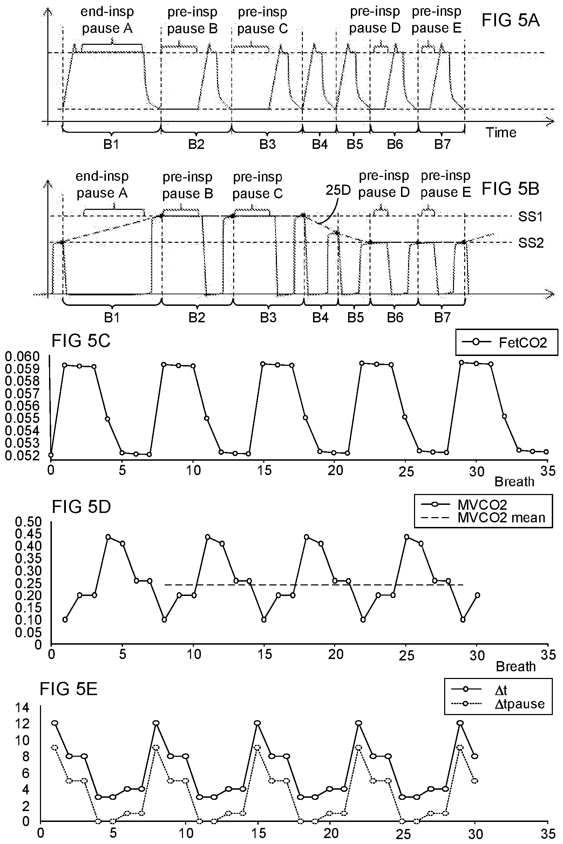

Figs. 5A-5E illustrate yet another exemplary ventilation pattern in which the proposed technique of forced steady state is applied both in phases of decreased ventilation and phases of increased ventilation. In this embodiment, each cycle of the ventilation pattern comprises a total of seven breaths B1-B7, three of which are breaths of decreased ventilation and four of which are breaths of increased ventilation. All breaths in the cycles of the cyclic ventilation pattern are identical in volume and differ from each other only in duration, in a manner causing the level of expired $CO_2$ to assume substantially steady states both in phases of decreased ventilation and phases of increased ventilation.

The first three breaths B1-B3 of decreased ventilation are identical to the breaths B1-B3 in Fig. 4A. In the fourth breath B4, the pre-inspiratory pause C that was present in the third breath B3 is removed, thereby initiating the phase of increased ventilation by causing a decrease in the level of expired $CO_2$. In this scenario, the fourth breath B4 alone is not sufficient to cause the desired change in the level of expired $CO_2$ and, therefore, a fifth breath B5 being identical to the fourth breath B4 is delivered immediately following said fourth breath. The fifth breath B5, in combination with said fourth breath B4, generates said substantial change in the level of expired $CO_2$. After the fifth breath, a sixth and a seventh breath B6-B7 having pre-inspiratory pauses D and E which are prolonged compared to the (non-existing) pre-inspiratory pause of the fifth breath B5 are delivered to the patient to prevent further decrease in the level of expired $CO_2$, and to cause the level of expired $CO_2$ to remain a the substantially constant steady state level SS2.

The variation in the level of expired $CO_2$ is illustrated by the dashed line 25D in Fig. 5B, and further in Fig. 5C showing the FetCO2 curve obtained during a simulation in which a patient was ventilated using a cyclic ventilation pattern in which each cycle corresponds to the ventilation pattern in Fig. 5A. Simulation data is shown for a sequence of 30 breaths. Fig. 5D illustrates the minute elimination (MVCO2) and mean minute elimination (MVCO2 mean) of $CO_2$ of the patient during said sequence of breaths.

Fig. 5E visualizes the changes in effective ventilation of the exemplary ventilation pattern of Fig. 5A and indicates the duration in seconds, $\Delta t$, of each breath in the sequence of breaths, and the total duration in seconds, $\Delta t$pause, of the inspiratory pause (including both end-inspiratory and pre-inspiratory pauses) of each breath in the sequence of breaths. In this exemplary embodiment, the first breath B1 of decreased ventilation comprises no pre-inspiratory pause and an end-inspiratory pause of approximately nine seconds, the second and third breaths B2-B3 of decreased ventilation comprise a pre-inspiratory pause of approximately five seconds and no end-inspiratory pause, the fourth and fifth breaths B4-B5 causing the transition from decreased to increased ventilation comprise no pre-inspiratory pause and no end-expiratory pause, whereas the sixth and seventh breaths B6-B7, constituting the last breaths in the phase of increased ventilation, comprises a pre-inspiratory pause of approximately one second and no end-expiratory pause. The total duration of each cycle of the cyclic ventilation pattern is 42 seconds.

Above it has been described that the breathing apparatus 1 (Fig. 1) is configured to ventilate the patient 3 in a manner that enables the at least one physiological parameter related to the ELV, cardiac output, EPBF and/or the $CO_2$ content of venous blood of the ventilated patient to be accurately and reliably determined. Determination may be made by external units, e.g. by an external computer or a monitoring system configured to obtain flow and $CO_2$ measurements related to the ongoing ventilation of the patient. Preferably, the breathing apparatus 1 is configured to determine the at least one physiological parameter itself. The above described ventilation pattern allows said at least one physiological parameter to be non-invasively determined by the breathing apparatus 1 in a continuous manner, e.g. on a breath-by-breath basis.

With reference again made to Fig. 1, the breathing apparatus 1 may comprise at least one flow sensor 27 for measuring at least an expiratory flow of expiration gas exhaled by the subject, and at least one CO2 sensor 29 for measuring the CO2 content of at least the expiration gas exhaled by the subject. The control unit may be configured to determine said at least one physiological parameter from flow and CO2 measurements obtained during an analysed sequence of breaths during which the subject is ventilated using said ventilation pattern. Preferably, the flow and CO2 sensors 27, 29 are configured to measure also inspiratory flow and CO2 content.

In the illustrated embodiment, the flow sensor 27 and the CO2 sensor 29 form parts of a capnograph 31 configured for volumetric capnography measurements. The capnograph 31 is arranged in the proximity of the airways opening of the patient 3, namely in the common line 9 of the breathing circuit in which it is exposed to all gas exhaled and inhaled by the patient 3. The capnograph 31 is connected to the breathing apparatus 1 via a wired or wireless connection 33, and configured to transmit the flow and CO2 measurements to the ventilator for further processing by the processing unit 21 of the breathing apparatus. The breathing apparatus 1 is preferably configured to generate a volumetric capnogram 35 from the flow and CO2 measurements received from the capnograph 31, and, additionally, to display the volumetric capnogram 35 on a display 37 of the ventilator.

In one embodiment, the control unit 5 of the breathing apparatus 1 is configured to determine the at least one physiological parameter based on said flow and CO2 measurements using the following capnodynamic equation for a single-chamber lung model, which describes how the fraction of alveolar carbon dioxide ($F_ACO2$) varies from one breath to another:

$$ELV \cdot \left(F_ACO2^n - F_ACO2^{n-1}\right) = \Delta t^n \cdot EPBF \cdot \left(C_VCO2 - C_ACO2^n\right) - VTCO2^n \qquad \text{(eq. 3)}$$

where ELV is the effective lung volume for CO2 storage at end of expiration, $F_ACO2^n$ is the alveolar CO2 fraction in the lung at end of expiration n, $\Delta t^n$ is the duration of breath n, EPBF is the effective pulmonary blood flow, CvCO2 is the CO2 concentration in mixed venous blood (volume of CO2 gas per volume blood), $C_ACO2^n$ is the CO2 concentration in alveolar capillaries during breath n, and $VTCO2^n$ is the tidal elimination of CO2 in breath n.

$F_ACO2^n$ may be measured by the CO2 sensor 29 while $C_ACO2^n$ and VTCO2 may be directly calculated from $F_ACO2^n$, the tidal volume of breath n ($VT^n$), and a known deadspace volume, as well known in the art, leaving EPBF, CvCO2 and ELV as unknown physiological parameters to be determined.

During steady state of expired CO2, the factor ($F_ACO2^n - F_ACO2^{n-1}$) in equation 3 becomes zero, allowing EPBF and CvCO2 to be determined independently of ELV in accordance with the principles described herein.

Equation 3 is analogous to equation 1 in WO2013/141766, disclosing a non-invasive and continuous method for simultaneous determination of ELV, cardiac output and CvCO2. Preferably, the control unit 5 of the breathing apparatus 1 is configured to use the method disclosed in WO2013/141766 to determine the parameter triplet {ELV, EPBF, CvCO2} from an analysed sequence of breaths, based on the correlation between the directly measureable or derivable parameters $\Delta F_ACO2$ (=$F_ACO2^n - F_ACO2^{n-1}$), $C_ACO2$ and VTCO2 in said analysed sequence of breaths. Of course, in exact correspondence with the method disclosed in WO2013/141766, the control unit 5 may also be configured to determine the parameter triplet {ELV, Q, CvCO2} based on the correlation between the directly measureable or derivable parameters $\Delta F_ACO2$, CaCO2 and VTCO2 in said analysed sequence of breaths. Here Q is the cardiac output, $\Delta F_ACO2$ is the change in volume fraction of alveolar CO2 between breath n and n-1, CaCO2 is the CO2 content of arterial blood, and VTCO2 is the tidal elimination of CO2. As mentioned in the background portion, EBPF is directly derivable from cardiac output, and vice versa (see eq. 2). Introducing an index 'n' indicating the number of the breath in the analysed sequence of breaths, and rearranging equation 3 such that the unknown parameters are gathered on the left-hand side of the equation yields:

$$ELV \cdot \Delta F_ACO_2{}^n - EPBF \cdot C_VCO_2 \cdot \Delta t^n + EPBF \cdot C_ACO_2^n \cdot \Delta t^n = -VTCO_2^n \qquad \text{(eq. 4)}$$

Writing this equation in matrix form for the breaths n=1, 2,...,N in the analysed sequence of breaths:

$$\begin{bmatrix} \Delta F_ACO_2^1 & -\Delta t^1 & C_ACO_2^1 \cdot \Delta t^1 \\ \vdots & \vdots & \vdots \\ \Delta F_ACO_2^n & -\Delta t^n & C_ACO_2^n \cdot \Delta t^n \\ \vdots & \vdots & \vdots \\ \Delta F_ACO_2^N & -\Delta t^N & C_ACO_2^N \cdot \Delta t^N \end{bmatrix} \cdot \begin{bmatrix} ELV \\ EPBF \cdot CvCO_2 \\ EPBF \end{bmatrix} = \begin{bmatrix} -VTCO_2^1 \\ \vdots \\ -VTCO_2^n \\ \vdots \\ -VTCO_2^N \end{bmatrix} \qquad \text{(eq.5)}$$

When the analysed sequence of breaths N comprises more than three breaths (i.e when N>3), this becomes an overdetermined system of equations and the unknown parameter triplet {ELV, EPBF·CvCO$_2$, EPBF} and hence the physiological parameters ELV, EPBF, and CvCO$_2$ can be determined by finding an approximate solution to the overdetermined system of equation. As well known in the art, the approximate solution to an overdetermined system of equations can be calculated in different ways, e.g. using the method of least squares. The solution to the overdetermined system of equations will depend on the correlation of the parameters $\Delta F_A CO2$, $C_A CO2$ and VTCO2 in the respiratory cycles of the analyses sequence of respiratory cycles.

This system of equations (eq. 5) may be rewritten as $A \cdot x_A = a$, where

$$A = \begin{bmatrix} \Delta F_A CO_2^1 & -\Delta t^1 & C_A CO_2^1 \cdot \Delta t^1 \\ \vdots & \vdots & \vdots \\ \Delta F_A CO_2^n & -\Delta t^n & C_A CO_2^n \cdot \Delta t^n \\ \vdots & \vdots & \vdots \\ \Delta F_A CO_2^N & -\Delta t^N & C_A CO_2^N \cdot \Delta t^N \end{bmatrix}, \; x_A = \begin{bmatrix} ELV \\ EPBF \cdot CvCO_2 \\ EPBF \end{bmatrix}, \text{ and } a = \begin{bmatrix} -VTCO_2^1 \\ \vdots \\ -VTCO_2^n \\ \vdots \\ -VTCO_2^N \end{bmatrix}$$

The control unit 5 of the breathing apparatus 5 may for example be configured to calculate an approximate solution for the parameter triplet {ELV, EPBF·CvCO$_2$, EBBF} by minimizing the error $|A \cdot x_A - a|$. Using the method of least squares, the solution may be calculated as:

$$x_A = \left( A^T \cdot A \right)^{-1} \cdot A^T \cdot a \qquad \qquad (eq. 6)$$

Consequently, the control unit 5 may determine approximate values of ELV, EPBF, CvCO2, and cardiac output from flow and CO2 measurements obtained for an analysed sequence of breaths during which the patient 3 is ventilated using a ventilation pattern causing the level of expired CO2 to vary during said analysed sequence of breaths. For continuous monitoring of ELV, EPBF, cardiac output and/or CvCO2, the ventilation pattern is preferably a cyclic ventilation pattern and the parameters are preferably determined by the control unit 5 on a breath-by-breath basis. Preferably but not necessarily, the number of breaths in said analysed sequence of breaths corresponds to the number of breaths in each cycle of the cyclic ventilation pattern.

Preferably, to allow EPBF, cardiac output and CvCO2 to be determined independently of ELV in accordance with the principles of the present invention, the breathing apparatus 1 is configured to ventilate the patient 3 using any of the above described ventilation patterns, and the control unit 5 is configured to determine EPBF, cardiac output and/or CvCO2 only from breaths during which the level of expired CO2 assumes a substantially steady state, or to determine EPBF, cardiac output and/or CvCO2 from a sequence of breaths in which breaths of substantially steady state are weighted more heavily than breaths of non-steady state. Once EPBF, cardiac output and/or CvCO2 has been determined, the control unit 5 may determine ELV only from transient breaths in said sequence of analysed breaths, or from a sequence of breaths in which transient breaths are weighted more heavily than breaths of steady state, preferably using the determined values of EPBF, cardiac output and/or CvCO2.

For example, the breathing apparatus 1 may be configured to ventilate the patient 3 using a cyclic ventilation pattern in which each cycle corresponds to the ventilation pattern shown in Fig. 3A, i.e. a cyclic ventilation pattern comprising alternating phases of three breaths of decreased ventilation (B1-B3 in Fig. 3A) and three breaths of increased ventilation (B4-B6 in Fig. 3A). For each breath n, the control unit 5 determines $\Delta F_A CO2$ ($F_A CO2^n - F_A CO2^{n-1}$), $C_A CO2$ and VTCO2 from known and measured parameters, and inserts the values of $\Delta F_A CO2$, $\Delta t$, $C_A CO2$ and VTCO2 into equation 5. After one cycle of the cyclic ventilation pattern, the following system of six equations is obtained, wherein equation 1 and 4 (n=1, 4) originate from transient breaths and equations 2-3 and 5-6 (n=2, 3, 5, 6) originate from breaths of substantially steady state.

$$\begin{bmatrix} \Delta F_A CO_2^1 & -\Delta t^1 & C_A CO_2^1 \cdot \Delta t^1 \\ \Delta F_A CO_2^2 & -\Delta t^2 & C_A CO_2^2 \cdot \Delta t^2 \\ \Delta F_A CO_2^3 & -\Delta t^3 & C_A CO_2^3 \cdot \Delta t^3 \\ \Delta F_A CO_2^4 & -\Delta t^4 & C_A CO_2^4 \cdot \Delta t^4 \\ \Delta F_A CO_2^5 & -\Delta t^5 & C_A CO_2^5 \cdot \Delta t^5 \\ \Delta F_A CO_2^6 & -\Delta t^6 & C_A CO_2^6 \cdot \Delta t^6 \end{bmatrix} \cdot \begin{bmatrix} ELV \\ EPBF \cdot CvCO_2 \\ EPBF \end{bmatrix} = \begin{bmatrix} -VTCO_2^1 \\ -VTCO_2^2 \\ -VTCO_2^3 \\ -VTCO_2^4 \\ -VTCO_2^5 \\ -VTCO_2^6 \end{bmatrix} \qquad (eq.7)$$

The control unit 5 may, in this scenario, be configured to determine EPBF and CvCO2 independently of ELV by calculating EPBF and CvCO2 only from equations 2, 3, 5 and 6 originating from breaths of steady state. The thus determined values of EPBF and CvCO2 may then be inserted into the system of equations, whereupon said system of equations can be solved by the control unit 5 with regard to ELV.

For each breath, the equation originating from the oldest breath in the analysed sequence of breath may be replaced by an equation originating from the most recent breath, whereby ELV, EPBF, cardiac output and CvCO2 can be monitored continuously by performing the above calculations on a breath-by-breath basis.

The ventilation pattern described herein may be predetermined, meaning that the ventilation pattern is determined prior to application thereof to the ventilated patient and does not alter or change in response to measured parameters. In other embodiments, the ventilation pattern may be an adaptive ventilation pattern which is automatically adapted based on measured parameters indicative of the response from the patient to the currently applied ventilation pattern. For example, the control unit 5 of the breathing apparatus 1 may be configured to use a measure of expired CO2, e.g. measured by the CO2 sensor 29, as control parameter for feedback control of the duration and/or volume of the breaths of the ventilation pattern. Thus, the control unit 5 may be configured to use expired CO2 for feedback control of the duration of the inspiratory pause (end-inspiratory and/or pre-inspiratory pauses) and/or the tidal volume of breaths in the ventilation pattern in order to effectuate the substantial change in the level of expired CO2 in the transition between phases of increased ventilation and decreased ventilation, and/or to cause the level of expired CO2 to assume a substantially steady state within the phase of increased and/or decreased ventilation. To this end, the control unit 5 may for example be configured to compare the level of expired CO2 in said at least first breath with the level of expired CO2 in said at least second breath, and, if the level of expired CO2 in said at least second breath deviates from the level of expired CO2 in said at least first breath by more than a predetermined amount, cause delivery of at least a third breath being different in duration and/or volume than said at least first breath and said at least second breath, which third breath is adapted to cause the level of expired CO2 to assume a substantially steady state during at least two breaths of the current phase, e.g. during said second and third breath.

Preferably, expired CO2 is measured even if not used as control parameter or for calculation of the at least one physiological parameter. This allows the breathing apparatus 1 to verify that a substantially steady state of expired CO2 is reached within the phases of increased and/or decreased ventilation, and thus to verify that the currently applied ventilation pattern really allows EPBF, cardiac output and/or CvCO2 to be determined independently of ELV. The control unit 5 of the breathing apparatus 1 may be configured to establish whether or not a substantially steady state is reached within a phase of increased or decreased ventilation by comparing CO2 measurements obtained during breaths of said phase of increased or decreased ventilation. If a substantially steady state is not reached, the control unit 5 may be configured to switch to another ventilation pattern hopefully capable of causing the level of expired CO2 to reach a steady state, and/or to trigger an alarm to an operator of the breathing apparatus. Furthermore, the control unit 5 may be configured to cause display of a curve illustrating variations in the level of expired CO2 over time on the display 37 of the breathing apparatus, for example a FetCO2 curve derivable from measurements obtained by the capnograph 31.

## Claims

1. A computer program for determination of at least one physiological parameter related to the ELV, cardiac output, EPBF and/or the CO2 content of venous blood of a subject (3) from flow and CO2 measurements obtained during mechanical ventilation of said subject by means of a breathing apparatus (1), the computer program comprising computer readable code which, when executed by a processing unit (21) of the breathing apparatus, causes the breathing apparatus to

   ventilate the subject using a ventilation pattern comprising at least one phase of decreased ventilation and at least one phase of increased ventilation, wherein each of the phase of decreased ventilation and the phase of increased ventilation comprises at least two breaths during which a level of CO2 expired by said subject assumes a substantially steady state (SS1, SS2), and
   determine the at least one physiological parameter from flow and CO2 measurements obtained during an analysed sequence of breaths during which the subject (3) is ventilated using said ventilation pattern,
   effectuate a first change in effective ventilation of the subject in order to initiate a phase of decreased or increased ventilation comprising at least a first breath which differs in duration and/or volume from a preceding breath, for generating a substantial change in the level of expired CO2 compared to the preceding breath, wherein the substantial change in the level of expired CO2, when measured as a fraction of CO2 in expired gas, is at least 0,3 percentage units,
   **characterised in that** the code, when executed by said processing unit (21), further causes the breathing apparatus to

effectuate a second change in effective ventilation of the subject within said phase of increased or decreased ventilation, wherein the second change in effective ventilation causes the phase to comprise at least a second breath that is different in duration and/or volume than said first breath and adapted to cause the level of expired CO2 to assume a substantially steady state during at least two consecutive breaths of said phase of decreased or increased ventilation, such that a level of expired CO2, when measured as a fraction of CO2 in expired gas, does not deviate by more than 0,1 percentage units between the consecutive breaths.

2. The computer program of claim 1, wherein both said phases of decreased and increased ventilation comprise at least a first breath for generating a substantial change in the level of expired CO2 compared to a preceding breath, and at least a second breath being different in duration and/or volume than said first breath, for causing the level of expired CO2 to assume said substantially steady state (SS1, SS2).

3. The computer program of claim 1 or 2, wherein said at least first breath for generating the substantial change in the level of expired CO2 is one single breath.

4. The computer program of any of the preceding claims, wherein said at least first and said at least second breath differ from a respective preceding breath in at least one of a duration of a pre-inspiratory pause, a duration of an end-inspiratory pause, and a tidal volume.

5. The computer program of any of the preceding claims, wherein the code, when executed by the processing unit (21) of the breathing apparatus (1), causes the breathing apparatus (1) to perform the steps of measuring expired CO2 in expiration gases expired by said subject (3), and using expired CO2 as control parameter for controlling the duration and/or volume of said at least second breath so as to obtain said substantially steady state level (SS1, SS2) of expired CO2.

6. The computer program of any of the preceding claims, wherein the code, when executed by the processing unit (21) of the breathing apparatus (1), causes the breathing apparatus (1) to perform the steps of:

- determining EPBF, cardiac output and/or the CO2 content of venous blood from a sequence of breaths comprising at least two breaths of substantially steady state within a phase of decreased ventilation and at least two breaths of substantially steady state within a phase of increased ventilation, and/or
- determining ELV from a sequence of breaths comprising at least two transient breaths between a phase of increased ventilation and a phase of decreased ventilation, or vice versa.

7. The computer program of any of the preceding claims, wherein the total number of breaths in the phase of decreased ventilation and the phase of increased ventilation is 4-10, preferably 5-8, and most preferably 6-7.

8. The computer program of any of the preceding claims, wherein the ventilation pattern is a cyclic ventilation pattern comprising a plurality of alternating phases of decreased and increased ventilation.

9. A breathing apparatus (1) for determination of at least one physiological parameter related to the ELV, cardiac output, EPBF and/or the CO2 content of venous blood of a subject (3) from flow and CO2 measurements obtained during mechanical ventilation of the subject by means of said breathing apparatus (1), comprising a control unit (5) configured to

control the operation of the breathing apparatus such that the subject is ventilated using a ventilation pattern comprising at least one phase of decreased ventilation and at least one phase of increased ventilation, each of the phase of decreased ventilation and the phase of increased ventilation comprising at least two breaths during which a level of CO2 expired by said subject (3) assumes a substantially steady state (SS1, SS2), and to determine the at least one physiological parameter from flow and CO2 measurements obtained during an analysed sequence of breaths during which the subject (3) is ventilated using said ventilation pattern, wherein the control unit (5) is configured to effectuate a first change in effective ventilation of the subject in order to initiate a phase of decreased or increased ventilation comprising at least a first breath which differs in duration and/or volume from a preceding breath, for generating a substantial change in the level of expired CO2 compared to the preceding breath, wherein the substantial change in the level of expired CO2, when measured as a fraction of CO2 in expired gas, is at least 0,3 percentage units, **characterised in that** the control unit (5) is further configured to effectuate a second change in effective ventilation of the subject within said phase of increased or decreased ventilation, wherein the second change in effective ventilation causes the phase to comprise at least a second breath that is different in duration and/or volume than said first breath and adapted to cause the level of expired CO2 to assume a substantially steady state during at least two consecutive breaths of said phase of decreased or increased ventilation, such that a level of expired CO2, when measured as a fraction of CO2 in expired gas, does not deviate by more than 0,1 percentage units between the consecutive breaths.

10. The breathing apparatus (1) of claim 9, wherein the control unit (5) is configured to cause both of said phases of decreased and increased ventilation to comprise at least a first breath for generating a substantial change in the level of expired CO2 compared to a preceding breath, and at least a second breath being different in duration and/or volume than said first breath, for causing the level of expired CO2 to assume said substantially steady state (SS1, SS2).

11. The breathing apparatus (1) of claim 9 or 10, wherein the control unit (5) is configured to cause said at least first breath to be one single breath.

12. The breathing apparatus (1) of any of the claims 9-11, wherein the control unit (5) is configured to cause said at least first and said at least second breath to differ from a respective preceding breath in at least one of a duration of a pre-inspiratory pause, a duration of an end-inspiratory pause, and a tidal volume.

13. The breathing apparatus (1) of any of the claims 9-12, comprising a CO2 sensor (29) for measuring expired CO2 in expiration gas expired by said subject (3), wherein the control unit (5) is configured to use expired CO2 as control parameter for controlling the duration and/or volume of said at least second breath so obtain said substantially steady state level (SS1, SS2) of expired CO2.

14. The breathing apparatus (1) of any of the claims 9-13, comprising a CO2 sensor (29) for measuring expired CO2 in expiration gas expired by said subject (3), and a flow sensor (27) for measuring the flow of said expiration gas, the control unit (5) being configured to:

- determine EPBF, cardiac output and/or the CO2 content of venous blood from a sequence of breaths comprising at least two breaths of substantially steady state within a phase of decreased ventilation and at least two breaths of substantially steady state within a phase of increased ventilation, and/or
- determine ELV from a sequence of breaths comprising at least two transient breaths between a phase of increased ventilation and a phase of decreased ventilation, or vice versa.

**Patentansprüche**

1. Computerprogramm zur Bestimmung mindestens eines physiologischen Parameters, der mit dem ELV, dem Herzzeitvolumen, dem EPBF und/oder dem CO2-Gehalt von venösem Blut eines Subjekts (3) in Bezug steht, anhand von Durchfluss- und CO2-Messungen, die während einer mechanischen Beatmung des Subjekts mittels einer Beatmungsvorrichtung (1) erlangt wurden, wobei das Computerprogramm computerlesbaren Code umfasst, der, wenn er von einer Verarbeitungseinheit (21) der Beatmungsvorrichtung ausgeführt wird, die Beatmungsvorrichtung zu Folgendem veranlasst:

Beatmen des Subjekts unter Verwendung eines Beatmungsmusters, umfassend mindestens eine Phase verringerter Beatmung und mindestens eine Phase verstärkter Beatmung, wobei jede der Phase verringerter Beatmung und die Phase verstärkter Beatmung mindestens zwei Atemzüge umfasst, während deren ein CO2-Spiegel, der von dem Subjekt ausgeatmet wird, einen im Wesentlichen stabilen Zustand (SS1, SS2) einnimmt, und Bestimmen des mindestens einen physiologischen Parameters anhand von Durchfluss- und CO2-Messungen, die während einer analysierten Sequenz von Atemzügen, während der das Subjekt (3) unter Verwendung des Beatmungsmusters beatmet wird, erlangt wurden,
Bewirken einer ersten Änderung in einer effektiven Beatmung des Subjekts, um eine Phase verringerter oder verstärkter Beatmung, umfassend mindestens einen ersten Atemzug, der sich in Dauer und/oder Volumen von einem vorhergehenden Atemzug unterscheidet, zu initiieren, um eine wesentliche Änderung in dem ausgeatmeten CO2-Spiegel im Vergleich zu dem vorhergehenden Atemzug zu erzeugen, wobei die wesentliche Änderung in dem ausgeatmeten CO2-Spiegel, wenn er als Anteil von CO2 in ausgeatmetem Gas gemessen wird, mindestens 0,3 Prozenteinheiten beträgt,
**dadurch gekennzeichnet, dass** der Code, wenn er von der Verarbeitungseinheit (21) ausgeführt wird, die Beatmungsvorrichtung ferner zu Folgendem veranlasst:

Bewirken einer zweiten Änderung in der effektiven Beatmung des Subjekts innerhalb der Phase verringerter oder verstärkter Beatmung, wobei die zweite Änderung der effektiven Beatmung die Phase veranlasst, mindestens einen zweiten Atemzug zu umfassen, der sich in Dauer und/oder Volumen von dem ersten Atemzug unterscheidet und dazu angepasst ist, den ausgeatmeten $CO_2$-Spiegel zu veranlassen, einen im Wesentlichen stabilen Zustand während mindestens zwei aufeinanderfolgenden Atemzügen der Phase verringerter oder verstärkter Beatmung einzunehmen, so dass ein ausgeatmeter $CO_2$-Spiegel, wenn er als Anteil von $CO_2$ in ausgeatmetem Gas gemessen wird, zwischen den aufeinanderfolgenden Atemzügen um nicht mehr als 0,1 Prozenteinheiten abweicht.

2. Computerprogramm nach Anspruch 1, wobei beide der Phasen verringerter und verstärkter Beatmung mindestens einen ersten Atemzug zum Erzeugen einer wesentlichen Änderung des ausgeatmeten $CO_2$-Spiegels im Vergleich zu einem vorhergehenden Atemzug und mindestens einen zweiten Atemzug, der sich in Dauer und/oder Volumen von dem ersten Atemzug unterscheidet, umfasst, um den ausgeatmeten $CO_2$-Spiegel zu veranlassen, den im Wesentlichen stabilen Zustand einzunehmen (SS1, SS2).

3. Computerprogramm nach Anspruch 1 oder 2, wobei der mindestens erste Atemzug zum Erzeugen der wesentlichen Änderung in dem ausgeatmeten $CO_2$-Spiegel ein einzelner Atemzug ist.

4. Computerprogramm nach einem der vorhergehenden Ansprüche, wobei der mindestens erste und der mindestens zweite Atemzug sich in mindestens einer von einer Dauer einer Pause vor dem Einatmen, einer Dauer einer Pause nach dem Einatmen und einem Atemzugvolumen von einem jeweiligen vorhergehenden Atemzug unterscheiden.

5. Computerprogramm nach einem der vorhergehenden Ansprüche, wobei der Code, wenn er von der Verarbeitungseinheit (21) der Beatmungsvorrichtung (1) ausgeführt wird, die Beatmungsvorrichtung (1) veranlasst, die Schritte des Messens von ausgeatmetem $CO_2$ in Ausatemgasen, die von dem Subjekt (3) ausgeatmet werden, durchzuführen und das ausgeatmete $CO_2$ als Steuerparameter zum Steuern der Dauer und/oder des Volumens des mindestens zweiten Atemzugs zu verwenden, um den im Wesentlichen stabilen Zustand (SS1, SS2) des ausgeatmeten $CO_2$-Spiegels zu erlangen.

6. Computerprogramm nach einem der vorhergehenden Ansprüche, wobei der Code, wenn er von der Verarbeitungseinheit (21) der Beatmungsvorrichtung (1) ausgeführt wird, die Beatmungsvorrichtung (1) veranlasst, die folgenden Schritte durchzuführen:

   - Bestimmen von EPBF, Herzzeitvolumen und/oder dem $CO_2$-Gehalt von venösem Blut anhand einer Sequenz von Atemzügen, umfassend mindestens zwei Atemzüge in dem im Wesentlichen stabilen Zustand innerhalb einer Phase verringerter Beatmung und mindestens zwei Atemzüge in dem im Wesentlichen stabilen Zustand innerhalb einer Phase verstärkter Beatmung, und/oder
   - Bestimmen des ELV anhand einer Sequenz von Atemzügen, umfassend mindestens zwei passagere Atemzüge zwischen einer Phase verstärkter Beatmung und einer Phase verringerter Beatmung oder umgekehrt.

7. Computerprogramm nach einem der vorhergehenden Ansprüche, wobei die Gesamtanzahl von Atemzügen in der Phase verringerter Beatmung und der Phase verstärkter Beatmung 4-10, bevorzugt 5-8 und am meisten bevorzugt 6-7 beträgt.

8. Computerprogramm nach einem der vorhergehenden Ansprüche, wobei es sich bei dem Beatmungsmuster um ein zyklisches Beatmungsmuster, umfassend eine Vielzahl abwechselnder Phasen verringerter und verstärkter Beatmung, handelt.

9. Beatmungsvorrichtung (1) zur Bestimmung mindestens eines physiologischen Parameters, der mit dem ELV, dem Herzzeitvolumen, dem EPBF und/oder dem $CO_2$-Gehalt von venösem Blut eines Subjekts (3) in Bezug steht, anhand von Durchfluss- und $CO_2$-Messungen, die während einer mechanischen Beatmung des Subjekts mittels der Beatmungsvorrichtung (1) erlangt wurden, umfassend eine Steuereinheit (5), die zu Folgendem konfiguriert ist:

   Steuern des Betriebs der Beatmungsvorrichtung derart, dass das Subjekt unter Verwendung eines Beatmungsmusters, umfassend mindestens eine Phase verringerter Beatmung und mindestens eine Phase verstärkter Beatmung, beatmet wird, wobei jede der Phase verringerter Beatmung und der Phase verstärkter Beatmung mindestens zwei Atemzüge umfasst, während deren ein $CO_2$-Spiegel, der von dem Subjekt (3) ausgeatmet wird, einen im Wesentlichen stabilen Zustand (SS1, SS2) einnimmt, und

Bestimmen des mindestens einen physiologischen Parameters anhand von Durchfluss- und $CO_2$-Messungen, die während einer analysierten Sequenz von Atemzügen, während der das Subjekt (3) unter Verwendung des Beatmungsmusters beatmet wird, erlangt wurden,

wobei die Steuereinheit (5) dazu konfiguriert ist, eine erste Änderung in einer effektiven Beatmung des Subjekts zu bewirken, um eine Phase verringerter oder verstärkter Beatmung, umfassend mindestens einen ersten Atemzug, der sich in Dauer und/oder Volumen von einem vorhergehenden Atemzug unterscheidet, zu initiieren, um eine wesentliche Änderung in dem ausgeatmeten $CO_2$-Spiegel im Vergleich zu dem vorhergehenden Atemzug zu erzeugen, wobei die wesentliche Änderung in dem ausgeatmeten $CO_2$-Spiegel, wenn er als Anteil von $CO_2$ in ausgeatmetem Gas gemessen wird, mindestens 0,3 Prozenteinheiten beträgt,

**dadurch gekennzeichnet, dass** die Steuereinheit (5) ferner dazu konfiguriert ist, eine zweite Änderung in der effektiven Beatmung des Subjekts innerhalb der Phase verstärkter oder verringerter Beatmung zu bewirken, wobei die zweite Änderung der effektiven Beatmung die Phase veranlasst, mindestens einen zweiten Atemzug zu umfassen, der sich in Dauer und/oder Volumen von dem ersten Atemzug unterscheidet und dazu angepasst ist, den ausgeatmeten $CO_2$-Spiegel zu veranlassen, einen im Wesentlichen stabilen Zustand während mindestens zwei aufeinanderfolgenden Atemzügen der Phase verringerter oder verstärkter Beatmung einzunehmen, so dass ein ausgeatmeter $CO_2$-Spiegel, wenn er als Anteil von $CO_2$ in ausgeatmetem Gas gemessen wird, zwischen den aufeinanderfolgenden Atemzügen um nicht mehr als 0,1 Prozenteinheiten abweicht.

10. Beatmungsvorrichtung (1) nach Anspruch 9, wobei die Steuereinheit (5) dazu konfiguriert ist, beide der Phasen verringerter und verstärkter Beatmung zu veranlassen, mindestens einen ersten Atemzug zum Erzeugen einer wesentlichen Änderung des ausgeatmeten $CO_2$-Spiegels im Vergleich zu einem vorhergehenden Atemzug und mindestens einen zweiten Atemzug, der sich in Dauer und/oder Volumen von dem ersten Atemzug unterscheidet, zu umfassen, um den ausgeatmeten $CO_2$-Spiegel zu veranlassen, den im Wesentlichen stabilen Zustand einzunehmen (SS1, SS2).

11. Beatmungsvorrichtung (1) nach Anspruch 9 oder 10, wobei die Steuereinheit (5) dazu konfiguriert ist, den mindestens ersten Atemzug zu veranlassen, ein einzelner Atemzug zu sein.

12. Beatmungsvorrichtung (1) nach einem der Ansprüche 9-11, wobei die Steuereinheit (5) dazu konfiguriert ist, den mindestens ersten und den mindestens zweiten Atemzug zu veranlassen, sich in mindestens einer von einer Dauer einer Pause vor dem Einatmen, einer Dauer einer Pause nach dem Einatmen und einem Atemzugvolumen von einem jeweiligen vorhergehendem Atemzug zu unterscheiden.

13. Beatmungsvorrichtung (1) nach einem der Ansprüche 9-12, umfassend einen $CO_2$-Sensor (29) zum Messen von ausgeatmetem $CO_2$ in Ausatemgas, das von dem Subjekt (3) ausgeatmet wurde, wobei die Steuereinheit (5) dazu konfiguriert ist, das ausgeatmete $CO_2$ als Steuerparameter zum Steuern der Dauer und/oder des Volumens des mindestens zweiten Atemzugs zu verwenden, um den im Wesentlichen stabilen Zustand (SS1, SS2) des ausgeatmeten $CO_2$-Spiegels zu erlangen.

14. Beatmungsvorrichtung (1) nach einem der Ansprüche 9-13, umfassend einen $CO_2$-Sensor (29) zum Messen von ausgeatmetem $CO_2$ in Ausatemgas, das von dem Subjekt (3) ausgeatmet wurde, und einen Durchflusssensor (27) zum Messen des Durchflusses des Ausatemgases, wobei die Steuereinheit (5) zu Folgendem konfiguriert ist:

   - Bestimmen von EPBF, Herzzeitvolumen und/oder dem $CO_2$-Gehalt von venösem Blut anhand einer Sequenz von Atemzügen, umfassend mindestens zwei Atemzüge in dem im Wesentlichen stabilen Zustand innerhalb einer Phase verringerter Beatmung und mindestens zwei Atemzüge in dem im Wesentlichen stabilen Zustand innerhalb einer Phase verstärkter Beatmung, und/oder
   - Bestimmen des ELV anhand einer Sequenz von Atemzügen, umfassend mindestens zwei passagere Atemzüge zwischen einer Phase verstärkter Beatmung und einer Phase verringerter Beatmung oder umgekehrt.

## Revendications

1. Programme informatique pour la détermination d'au moins un paramètre physiologique lié à l'ELV, au débit cardiaque, à l'EPBF et/ou à la teneur en $CO_2$ de sang veineux d'un sujet (3) à partir de mesures d'écoulement et de $CO_2$ obtenues pendant la ventilation mécanique dudit sujet au moyen d'un appareil respiratoire (1), le programme informatique comprenant un code lisible par ordinateur qui, lorsqu'il est exécuté par une unité de traitement (21) de l'appareil respiratoire, amène l'appareil respiratoire à

ventiler le sujet en utilisant un profil de ventilation comprenant au moins une phase de ventilation diminuée et au moins une phase de ventilation augmentée, dans lequel chacune de la phase de ventilation diminuée et de la phase de ventilation augmentée comprend au moins deux respirations pendant lesquelles un niveau de $CO_2$ expiré par ledit sujet adopte un état sensiblement stable (SS1, SS2), et

déterminer l'au moins un paramètre physiologique à partir de mesures d'écoulement et de $CO_2$ obtenues pendant une séquence analysée de respirations pendant laquelle le sujet (3) est ventilé en utilisant ledit profil de ventilation,

effectuer un premier changement de ventilation effective du sujet afin d'initier une phase de ventilation diminuée ou augmentée comprenant au moins une première respiration qui diffère en durée et/ou en volume d'une respiration précédente, pour générer un changement substantiel du niveau de $CO_2$ expiré par rapport à la respiration précédente, dans lequel le changement substantiel du niveau de $CO_2$ expiré, lorsqu'il est mesuré en tant que fraction de $CO_2$ dans le gaz expiré, est d'au moins 0,3 unité de pourcentage,

**caractérisé en ce que** le code, lorsqu'il est exécuté par ladite unité de traitement (21), amène en outre l'appareil respiratoire à

effectuer un second changement de ventilation efficace du sujet dans ladite phase de ventilation augmentée ou diminuée, dans lequel le second changement de ventilation efficace amène la phase à comprendre au moins une seconde respiration qui est différente en durée et/ou en volume de ladite première respiration et conçue pour amener le niveau de $CO_2$ expiré à adopter un état sensiblement stable pendant au moins deux respirations consécutives de ladite phase de ventilation diminuée ou augmentée, de sorte qu'un niveau de $CO_2$ expiré, lorsqu'il est mesuré en tant que fraction de $CO_2$ dans le gaz expiré, ne s'écarte pas de plus de 0,1 unité de pourcentage entre les respirations consécutives.

2. Programme informatique selon la revendication 1, dans lequel les deux dites phases de ventilation diminuée et augmentée comprennent au moins une première respiration permettant la génération d'un changement substantiel du niveau de $CO_2$ expiré par rapport à une respiration précédente, et au moins une seconde respiration étant différente en durée et/ou en volume de ladite la première respiration, permettant d'amener le niveau de $CO_2$ expiré à adopter ledit état sensiblement stable (SS1, SS2).

3. Programme informatique selon la revendication 1 ou 2, dans lequel ladite au moins première respiration permettant la génération du changement substantiel du niveau de $CO_2$ expiré est une seule respiration.

4. Programme informatique selon l'une quelconque des revendications précédentes, dans lequel ladite au moins première respiration et ladite au moins seconde respiration diffèrent d'une respiration précédente respective dans au moins un élément parmi une durée d'une pause pré-inspiratoire, une durée d'une pause de fin d'inspiration et un volume courant.

5. Programme informatique selon l'une quelconque des revendications précédentes, dans lequel le code, lorsqu'il est exécuté par l'unité de traitement (21) de l'appareil respiratoire (1), amène l'appareil respiratoire (1) à réaliser les étapes de mesure de $CO_2$ expiré dans les gaz d'expiration expirés par ledit sujet (3), et d'utilisation de $CO_2$ expiré comme paramètre de commande pour commander la durée et/ou le volume de ladite au moins seconde respiration de manière à obtenir ledit niveau d'état sensiblement stable (SS1, SS2) de $CO_2$ expiré.

6. Programme informatique selon l'une quelconque des revendications précédentes, dans lequel le code, lorsqu'il est exécuté par l'unité de traitement (21) de l'appareil respiratoire (1), amène l'appareil respiratoire (1) à réaliser les étapes consistant à :

- déterminer l'EPBF, le débit cardiaque et/ou la teneur en $CO_2$ du sang veineux à partir d'une séquence de respirations comprenant au moins deux respirations à l'état sensiblement stable dans une phase de ventilation diminuée et au moins deux respirations à l'état sensiblement stable dans une phase de ventilation augmentée, et/ou
- déterminer l'ELV à partir d'une séquence de respirations comprenant au moins deux respirations transitoires entre une phase de ventilation augmentée et une phase de ventilation diminuée, ou vice versa.

7. Programme informatique selon l'une quelconque des revendications précédentes, dans lequel le nombre total de respirations dans la phase de ventilation diminuée et la phase de ventilation augmentée est de 4 à 10, de préférence de 5 à 8, et de préférence encore de 6 à 7.

8. Programme informatique selon l'une quelconque des revendications précédentes, dans lequel le profil de ventilation est un profil de ventilation cyclique comprenant une pluralité de phases alternées de ventilation diminuée et augmentée.

9. Appareil respiratoire (1) pour la détermination d'au moins un paramètre physiologique lié à l'ELV, au débit cardiaque, à l'EPBF et/ou à la teneur en $CO_2$ du sang veineux d'un sujet (3) à partir de mesures d'écoulement et de $CO_2$ obtenues pendant la ventilation mécanique du sujet au moyen dudit appareil respiratoire (1), comprenant une unité de commande (5) configurée pour

commander le fonctionnement de l'appareil respiratoire de sorte que le sujet soit ventilé en utilisant un profil de ventilation comprenant au moins une phase de ventilation diminuée et au moins une phase de ventilation augmentée, chacune de la phase de ventilation diminuée et de la phase de ventilation augmentée comprenant au moins deux respirations pendant lesquelles un niveau de $CO_2$ expiré par ledit sujet (3) adopte un état sensiblement stable (SS1, SS2), et pour
déterminer l'au moins un paramètre physiologique à partir de mesures d'écoulement et de $CO_2$ obtenues pendant une séquence analysée de respirations pendant laquelle le sujet (3) est ventilé en utilisant ledit profil de ventilation,
dans lequel l'unité de commande (5) est configurée pour effectuer un premier changement de ventilation effective du sujet afin d'initier une phase de ventilation diminuée ou augmentée comprenant au moins une première respiration qui diffère en durée et/ou en volume d'une respiration précédente, permettant la génération d'un changement substantiel du niveau de $CO_2$ expiré par rapport à la respiration précédente, dans lequel le changement substantiel du niveau de $CO_2$ expiré, lorsqu'il est mesuré en tant que fraction de $CO_2$ dans le gaz expiré, est d'au moins 0,3 unité de pourcentage,
**caractérisé en ce que** l'unité de commande (5) est en outre configurée pour effectuer un second changement de ventilation efficace du sujet dans ladite phase de ventilation augmentée ou diminuée, dans lequel le second changement de ventilation efficace amène la phase à comprendre au moins une seconde respiration qui est différente en durée et/ou en volume de ladite première respiration et conçue pour amener le niveau de $CO_2$ expiré à adopter un état sensiblement stable pendant au moins deux respirations consécutives de ladite phase de ventilation diminuée ou augmentée, de sorte qu'un niveau de $CO_2$ expiré, lorsqu'il est mesuré en tant que fraction de $CO_2$ dans le gaz expiré, ne s'écarte pas de plus de 0,1 unité de pourcentage entre les respirations consécutives.

10. Appareil respiratoire (1) selon la revendication 9, dans lequel l'unité de commande (5) est configurée pour amener les deux dites phases de ventilation diminuée et augmentée à comprendre au moins une première respiration permettant la génération d'un changement substantiel du niveau de $CO_2$ expiré par rapport à une respiration précédente, et au moins une seconde respiration étant différente en durée et/ou en volume de ladite première respiration, pour amener le niveau de $CO_2$ expiré à adopter ledit état sensiblement stable (SS1, SS2).

11. Appareil respiratoire (1) selon la revendication 9 ou 10, dans lequel l'unité de commande (5) est configurée pour amener ladite au moins première respiration à être une seule respiration.

12. Appareil respiratoire (1) selon l'une quelconque des revendications 9 à 11, dans lequel l'unité de commande (5) est configurée pour amener ladite au moins première respiration et ladite au moins seconde respiration à différer d'une respiration précédente respective dans au moins un élément parmi une durée d'une pause pré-inspiratoire, une durée d'une pause de fin d'inspiration et un volume courant.

13. Appareil respiratoire (1) selon l'une quelconque des revendications 9 à 12, comprenant un capteur de $CO_2$ (29) permettant de mesurer le $CO_2$ expiré dans le gaz d'expiration expiré par ledit sujet (3), dans lequel l'unité de commande (5) est configurée pour utiliser le $CO_2$ expiré comme paramètre de commande pour commander la durée et/ou le volume de ladite au moins seconde respiration afin d'obtenir ledit niveau d'état sensiblement stable (SS1, SS2) de $CO_2$ expiré.

14. Appareil respiratoire (1) selon l'une quelconque des revendications 9 à 13, comprenant un capteur de $CO_2$ (29) permettant de mesurer le $CO_2$ expiré dans le gaz d'expiration expiré par ledit sujet (3), et un capteur de débit (27) pour mesurer le débit dudit gaz d'expiration, l'unité de commande (5) étant configurée pour :

- déterminer l'EPBF, le débit cardiaque et/ou la teneur en $CO_2$ du sang veineux à partir d'une séquence de respirations comprenant au moins deux respirations d'état sensiblement stable dans une phase de ventilation diminuée et au moins deux respirations d'état sensiblement stable dans une phase de ventilation augmentée, et/ou
- déterminer l'ELV à partir d'une séquence de respirations comprenant au moins deux respirations transitoires entre une phase de ventilation augmentée et une phase de ventilation diminuée, ou vice versa.

FIG 1

FIG 4A

FIG 4B

FIG 2A

FIG 2B

FIG 2C

FIG 2D

FIG 2E

FIG 3A

FIG 3B

FIG 3C

FIG 3D

FIG 5A

FIG 5B

FIG 5C

FIG 5D

FIG 5E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7135001 B, Orr
- WO 2006119546 A1
- WO 2006119546 A
- WO 2013141766 A, Emtell and Hallbäck
- EP 2799008 A, Emtell and Hallbäck
- US 2013345586 A1, FISHER

### Non-patent literature cited in the description

- **CAPEK, J M ; ROY, RJ**. Noninvasive measurement of cardiac output using partial CO2 rebreathing. *IEEE Trans. Biomed. Eng.*, 1988, vol. 35, 653-661
- **GAMA DE ABREU, M et al.** Partial carbon dioxide rebreathing: A reliable technique for noninvasive measurement of nonshunted pulmonary capillary blood flow. *Crit. Care Med.*, 1997, vol. 25, 675-683
- **PEYTON et al.** Noninvasive, automated and continuous cardiac output monitoring by pulmonary capnodynamics: breath-by-breath comparison with ultrasonic flow probe. *Anesthesiology*, July 2006, vol. 105 (1), 72-80